# EUROPEAN PATENT APPLICATION

(11) **EP 1 013 775 A1**
(43) Date of publication of application: **28.06.2000**
(21) Application number: 98124317.3
(22) Date of filing: 21.12.1998
(51) Int. Cl.: C12Q 1/68, C12Q 1/70, C07H 21/04

(54) **Quantitative polymerase chain reaction using a fluorogenic real-time detection system**

(71) Applicant: LUTZ, Hans, CH-8455 Rüdlingen (CH)
(72) Inventor: The designation of the inventor has not yet been filed
(74) Representative: Blum, Rudolf Emil Ernst

(57) **Abstract**

The present invention concerns an improved quantitative one-tube fluorogenic real time polymerase chain reaction. The improvement exists in the use of very specific probes allowing much more reliable and fast analysis, since they allow the selective analysis of various pathogens and mRNAs coding for cytokines and other proteins. The improvement is obtained by using probes selectively hybridizing with the pathogens of interest.

## Description

### Field of the invention

The present invention concerns the characterization of assay conditions and specific nucleotide sequence for use in a quantitative PCR with enhanced rapidity and sensitivity. In particular, the invention concerns an improved one tube fluorogenic real time PCR method suited for the detection of infectious agents, the quantitation of mRNA expression and the detection of genetic mutations.

### Background of the invention

To detect pathogens or pathogen-induced reactions and/or to study the efficacy of candidate vaccines or therapeutics, test systems are needed to quantify a specific target substance. Several PCR based methods have been proposed to quantify such target substances, e.g. proviruses and viruses. One of the most common concepts of quantitative competitive PCR (qcPCR) is the coamplification of two different templates of similar lengths and with the same primer recognition sequences in the same tube, thus ensuring optimised thermodynamics and amplification efficiencies for both template species (Menzo et al., 1992; Siebert and Larrick, 1992). Usually several dilutions of the competitor DNA are coamplified with the same amount of genomic DNA (or RNA in the case of RT-PCR). After amplification, products of both template species can be detected by different methods: ELOSA (Enzyme Linked Oligonucleotide Sorbent Assay) combines a probe hybridisation technique with a simple method of detecting and quantifying PCR products in a microtiter format (Adler et al., 1992). Southern hybridisation is sensitive (Southern, 1975) but requires multiple post-PCR steps and can take several days before quantitation is completed. In gel electrophoresis, the two targets must be clearly distinguishable to allow densitometric evaluation of the relative intensities of the bonds. Because the templates compete for amplification (and in the case of competitive RT-PCR, also for reverse transcription), any variable affecting amplification has the same effect on both. Around the equivalence point, the ratio of PCR products reflects the ratio between the initial amount of the two sequences allowing precise evaluation of the amount of wild-type template. Another approach is the end-point dilution PCR, a method that was among the first developed (Simmonds et al., 1990; Zhang et al., 1993). However, the described PCR approaches have several important drawbacks (Clementi et al., 1993): (i) In particular, the techniques require several amplification reactions per sample, thus, they are expensive and allow the analysis of only a few samples for each amplification run. (ii) The postamplification steps are time consuming and may lead to inaccuracy and contamination or carryover. (iii) Quantitation using end-point dilution assay narrows the linear range to three to four log decades (Sundfors and Collan 1996).

It is also already known to use a quantitative system comprising a dual-labeled fluorogenic probes e.g. described in WO 92/02638 the disclosure of which is incorporated herein by reference. Such dual-labeled fluorogenic probes are e.g. available as TaqMan™ probe (TaqMan™, Perkin-Elmer Corp./Applied Biosystems, Foster City, California; Holland et al., 1991). The fluorogenic 5' nuclease PCR assay takes advantage of the endogenous 5'-3' nuclease activity of polymerases such as Taq DNA polymerase to digest the TaqMan™ probe which hybridizes to the amplicon during PCR. When the probe is intact, the two fluorophores interact such that the emission of the reporter dye is quenched. Reporter and quencher in proximity results in suppression of reporter fluorescence of the intact probe by Förster-type energy transfer (Förster, 1948). Upon binding of the probe downstream during PCR, the 5'-3' nuclease activity of *Taq* DNA polymerase releases the reporter from the vicinity of the quencher dye resulting in increased reporter fluorescence. Fluorescence intensity is directly related to the amount of input target DNA and can be detected with an automated fluorometer. Quantitation is accomplished by comparison of fluorescence signals obtained from samples with unknown amounts of nucleotide sequence to be amplified with the fluorescence signals obtained from standard dilutions of the target substance.

Although the TaqMan™ method provides essential advantages over the usual quantitative PCR methods, due to the use of usual probes and primers it has several drawbacks. The reliability and security of diagnosis is often limited due to the fact that closely related mutations, although leading to the same or a very similar disease, often are not detected or similar mutations are detected although only some of them are pathogenic. Thus, the hitherto used method is only suitable for a limited number of targets.

It was therefore a first object of the present invention to provide an improved one-tube qcPCR method with increased reliability of the PCR reaction based diagnosis and application to a lot of pathogens hitherto only detectable and quantifiable with difficulties.

It was a further object of the present invention to provide primer sequences and probe sequences especially suitable for the use in such a one-tube qcPCR method.

It was still a further object to provide a method for the determination of suitable primer and probe sequences.

### Short description of the Figures

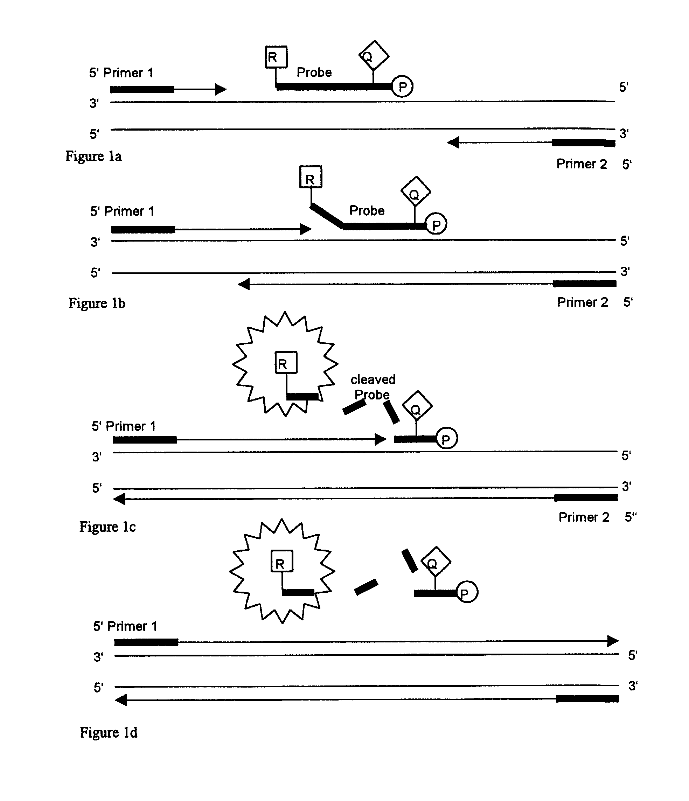

Figure 1 shows the principles of the 5'-3' nuclease fluorogenic assay with

Figure 1a showing the polymerase chain reaction (PCR) forward primer 1, the PCR reverse primer 2 and the fluorogenic probe labeled with a reporter dye R and a quencher dye Q and 3' phosphate group P, all annealed to the DNA template.

Figure 1b shows how upon polymerization the polymerase extends the primer and displaces the probe.

Figure 1 c shows the cleavage of the displaced probe resulting in an increase in relative fluorescence of the reporter.

Figure 1 d shows the completion of the polymerisation.

Figure 2 shows the position of primers and probe in the sequence of the FCoV 7b gene (SEQ ID No 85).

Figure 3 shows the conformity of the FCoV fluorogenic assay sequence (FCoVfa)with the sequence of canine coronavirus (CCV), transmissible gastroenteritis virus (TGEV), porcine respiratory coronavirus (PRCV), and human coronavirus 229E (HCV). The inconformity between the FcoVfa sequence and the HCV sequence decreases the risk of contamination due to shedded human coronaviruses. Figure 4 shows for FCoV the amplification efficiencies of standard RNA and complete virus. Δs is the difference in both slopes.

Figure 5 shows the positions of the two FIV gag-specific probes and corresponding primers within the FIV gag-sequence of FIV Petaluma (GenBank accession no M25381)(SEQ ID No 86).

Figure 6a shows the linearity of a dilution of standard molecules derived from FIV gag (see Fig. 5) over 8 log decades.

Figure 6b shows the amplification efficiency of target DNA and standard plasmid with probe FIV581p.

Figure 6c shows the results of a recovery experiment with standard plasmid spiked cell lysates and standard plasmid alone.

Figure 6d shows the influence of carrier DNA (calf thymus DNA) with FIV581p: 50 molecules of standard plasmids were amplified in a PCR method according to the present invention with varying amounts of carrier DNA (6000 µg/ml - 5 µg/ml).

Figure 7 shows the primer and probe sequences of Examples 3 to 26.

### Description of the invention

The goal of the present invention is met by a one-tube qcPCR method wherein PCR product accumulation is measured through at least one specific label such as a label-system, e.g. a dual-labeled fluorogenic probe, and whereby the probe as well as the primers are designed to meet specific requirements.

It was surprisingly found that the reliability of a one-tube qcPCR method can largely be enhanced if at least one very specific probe is used.

The at least one probe of the present invention must meet the following requirements.

It must hybridize with the target sequence prior to the complementary strand and prior to the primers. This is usually acchieved with a difference in the melting temperatures of about 5 to 6°C, preferably about 10°C.

It has now been found that diagnosis can be improved if the probe/primer relation does not only meet the above outlined temperature requirement but is additionally very specificly designed.

It is known that pathogens can exist in form of slightly different isolates or mutants, respectively, all of which induce the same disease. In order to recognize such a disease or a risk for the outbreak of such disease it is of great importance to detect all possible variants of the respective pathogen. Using a usual probe, very often several analyses have to be performed in order to get all relevant mutants. On the other hand, there exist very similar mutants of which only part acts as a pathogen in a specific organism. For such mutants it is of interest to distinguish between the pathogenic and the non-pathogenic mutants.

The specific probes of the present invention meet the following requirements:
- they anneal prior to the primers
- they have a length sufficiently long to assure hybridization or non-hybridization with sequences comprising altered nucleic acids due to mutants, different isolates etc. leading to mismatches, preferably a length of at least 20 nucleic acids and a hybridization force of at least 50, whereby the hybridization force is calculated as the sum of all hydrogen bonds, e.g. 3 bonds for each C or G and 2 bonds for each A or T,
- the length is dependent on the mismatches of mutants to be covered or eliminated, respectively, whereby the maximal hybridization force of the probe must be greater if sequences leading to more mismatches have to be detected, preferably the mismatches reduce the hybridization to an extent of not more but about 20 %, much preferably not more but about 15 %, whereby the minimal hybridization force has to be about 50.

Thus, for a probe with a length of 25 bp and a maximal hybridization force (no mismatches) of about 60, at most 3 mismatches are usually allowed, whereby a probe of only 20 bp length in general is very selective.

Of course, the skilled person recognizes that the hybridization force outlined above is a rough estimate only, however, it enables the selective design of probes with enhanced analytical properties.

It has furthermore been found that the detection is improved if the probe and primers lead to a total amplicon length of at most 300 bp, preferably of at most 350 bp, much preferred about 100 to about 225 bp. The minimal length possible must allow for the at least one nucleotide between the primer and the probe, and thus corresponds to the length of the two primers plus the length of the probe plus one further base. However, longer amplicons of at least about 100 bp are much preferred (see above).

It has been found that the short amplicon length preferred according to the present invention leads to several advantages such as very fast analysis cycles due to the fast termination of the amplification because of the shortness of the amplicon (the time needed for a full amplification is in the range of the heating time), and improved integrity (completeness) of the DNA sequence, therewith improving the reliability of the quantification of the pathogen.

In preferred embodiments also at least one to all of the following features are additionally present.
(a) The probe usually has a melting temperature Tₘ which is at least 6°C, preferably about 10°C higher than the one of the primers, preferably a Tₘ in the temperature range of 67 to 71°C, in particular about 69°C.
(b) The primers preferably have a melting temperature of from 57 to 61°C, in particular about 59°C.
(c) The primers preferably have a length of 18 to 22 bp and the probes of 24 to 30 bp, in order to cover several mutants or isolates, respectively, or of about 20 to 23 bp if a specific isolate or mutant shall be detected and quantified.
(d) The distance between primer and probe is at least about 1 bp, and for the above defined preferred amplicon length at most the length of the amplicon minus the length of the two primers and the probe, for an amplicon length of 300 bp, about 240 bp. However, preferably the distance is at most about 100 bp.
(e) The cycle preferably is performed with two temperatures, said temperatures preferably being the Tₘ of the primers and the denaturation temperature, whereby said denaturation temperature much preferably is from about 85 to about 95°C.
(f) The probe is selected from a region where an intron has been spliced off, whereby the probe comprises from 30 to 70 % of a first exon and from 70 to 30 % of a second exon, preferably about 50 % of each exon.

Below, the above outlined features are further described.
(a) The required melting temperature of the probe and the temperature difference between the primers and the probe of at least 6°C can be obtained by different measures. A higher Tₘ is obtained either if the probe is selected of a region rich in G and C, or if a longer probe is selected, e.g. in the upper range of the mentioned length range.
(b) Also for the primers adjustment of the melting temperature can be made by either using G and C rich sequences for a given length or longer sequences to enhance the melting temperature, or sequences poor in G and C, or shorter sequences, respectively, to lower the melting temperature. Also for the primers mutants have to be considered. They have to be selected from a highly conserved region and they must be sufficiently long or C and/or G rich to hybridize with sufficient security. In addition, the two primers of each amplification set should have a difference in melting temperature of at most 4°C in order to allow very fast heating and cooling operations without loss of sensitivity.
(c) A length of the primers in the range of from 18 to 22 bp and of the probes in a range of from at least 24 to 40 bp, in particular 24 to 30 bp, has turned out to be much preferable for analysis of different mutants since these lengths allow fast hybridization and melting under favorable conditions as well as hybridization to sequences that do not entirely match, whereby up to three mismatches for probes of 24 bp length are still acceptable, for the shorter primers no mismatches are allowed to occur within the last six nucleotides from the 3' end and not more than two mismatches within the remaining sequence.
(d) By the limited distance between the primer and the probe a fast signal is obtained since the polymerase has only a short sequence to synthesize until the probe is reached and a signal is obtained, e.g. in that the fluorescent marker is separated from the quencher. Although usually a longer distance is considered as preferable due to a longer time for the probe to bind, it has been found that with the method of the present invention no probe binding problems occur.

Beside of the fast signal obtainable by the limited distance between the primer and the probe fast analysis cycles and very good quantitation are obtained by limiting the whole length of the amplicon (see also above).

For most targets, sequences meeting the above mentioned requirements a) to d) can be found.

(f) It is a well recognized problem that the selective detection of pathogens is rendered difficult by the hardly avoidable presence of DNA, be it genomic DNA of the sample or DNA impurities imported from earlier analysis. If DNA impurities from former amplifications cannot be avoided, it is known to perform sterilization reactions, e.g. by selectively degrading DNA prior to reverse transcription of RNA (see e.g. EP 632 134). However, such sterilisation processes are very time consuming and often not satisfactory. In particular, they are not applicable for samples to be analysed which are themselves rich in genomic DNA (gDNA). The present invention thus provides an improved method for securing the exclusive amplification and quantification of mRNA derived cDNA. This improvement is achieved by selecting the probe in such a way that it spans two exons, whereby it has about the same number of bp from each exon. It was surprisingly found that probes of the length as disclosed herein readily hybridize with sequences where the intron interrupting the "probe sequence" is spliced off, i.e. cDNA sequences derived from mature mRNA, whereby for genomic DNA no loop formation and therefore also no hybridization was found.

The requirement that the probe is selected from two exons ensures that only intron free DNA is measured, i.e. cDNA derived from mRNA, and not also genomic DNA.

It is nevertheless preferred to avoid crosscontamination. It has been shown in the scope of the present invention that crosscontamination can largely be prevented by performing all pipetting in a laminar flow hood.

Thus, the present invention provides a method for the identification and quantification of at least one pathogen in a sample, said method being performed on the DNA level, and said method comprising:
(A) providing to a PCR assay containing said sample, at least one labeled oligonucleotide probe containing a sequence complementary to a region of the target nucleic acid, wherein said labeled oligonucleotide anneals within the target nucleic acid sequence bounded by the oligonucleotide primer of step (B);
(B) providing at least one set of oligonucleotide primers, wherein a first primer contains a sequence complementary to a region in one strand of the target nucleic acid sequence and primes the synthesis of a complementary DNA strand, and a second primer contains a sequence complementary to a region in a second strand of the target nucleic acid sequence and primes the synthesis of a complementary DNA strand; and wherein each oligonucleotide primer is selected to anneal to its complementary template upstream of any labeled oligonucleotide annealed to the same nucleic acid strand;
(C) amplifying the target nucleic acid sequence employing a nucleic acid polymerase having 5' to 3' nuclease activity as a template-dependent polymerizing agent under conditions which are permissive for PCR cycling steps of (i) annealing of primers and labeled oligonucleotide to a template nucleic acid sequence contained within the target region, and (ii) extending the primer, wherein said nucleic acid polymerase synthesizes a primer extension product while the 5' to 3' nuclease activity of the nucleic acid polymerase simultaneously releases labeled fragments from the annealed duplexes comprising labeled oligonucleotide and its complementary template nucleic acid sequences, thereby creating detectable labeled fragments; and
(D) detecting and/or measuring the release of labeled fragments to determine the presence or absence of target sequence in the sample, characterized in that at least one of said labeled oligonucleotide probe(s), preferably all of said probe(s), have a length and/or a content of G and/or C sufficient to provide a hybridization force stong enough to ensure hybridization in the presence of just the desired number of possible mismatches.

Preferably the length is at least about 20 nucleic acids and the minimal hybridization force is about 50, calculated as the sum of all hydrogen bonds, e.g. 3 bonds for each C or G and 2 bonds for each A or T.

The length of the probe is dependent on the mismatches of mutants to be covered or eliminated, respectively, whereby the hybridization force of the probe must be greater for more mismatches. Preferably the mismatches reduce the hybridization to an extent of not more but 20 %, much preferably not more but 15 %, whereby the minimal hybridization force was determined to be about 50.

In preferred embodiments one or several or all of the further requirements (a) to (f) outlined above are also met.

As used herein, a sample comprising a target can be any substance containing or presumed to contain a nucleic acid target and includes a sample of tissue or fluid isolated from an individual or individuals, including but not limited to, for example, skin, plasma, serum, spinal fluid, lymph fluid, synovial fluid, urine, tears, blood cells, organs, tumors, and also to samples of in vitro cell culture constituents (including but not limited to conditioned medium resulting from the growth of cells in cell culture medium, recombinant cells and cell components), and to material in the environment including but not limited to for example, plants, wood, rock, sand, etc. A set of primers refers to two primers, one for each strand of a double-stranded nucleic acid sequence.

Possible targets include double- and single-stranded DNA as well as single-stranded and low abandoned double-stranded RNA. Primers and probes preferably are DNA oligonucleotides, whereby specific nucleic acids can be present in one or both of the primers of a set and/or in a probe in order to provide them with specific features.

The primers and probes are not necessarily physically derived from any existing or natural sequence but may be generated in any manner, including chemical synthesis, DNA replication, reverse transcription, mutations such as site specific mutations or a combination of such methods. Thus, the primers and probes of the present invention can be designed or selected so that they allow much faster and more significant analysis since due to the specific probes mutants of one pathogen, all inducing the disease, can be simultaneously detected and mutants not causing a specific disease can be excluded from analysis.

By the specific probes of the present invention the number of needed probes can be reduced, thereby reducing the preparation time and costs.

Additionally, by the specific length of the amplicon preferred according to the present invention, the time for one analysis cycle is much reduced and the integrity of the DNA sequence is improved. Furthermore, although the limited amplicon length reduces the possible distance between the primer and the probe, the probes of the present invention readily bind leading to the desired signaling effect.

The melting temperature Tₘ of a particular nucleic acid duplex under specified conditions is the temperature at which half of the base pairs have disassociated.

As used herein, the term "target", "target sequence" or "target nucleic acid sequence" refers to a DNA or RNA sequence or a region thereof which is to be either amplified, detected or both. The target sequence resides between the two primer sequences used for amplification and can be a part of either one specific sequence to be distinguished from similar sequences or can include parts of several mutants of a specific pathogen.

As used herein, the term "probe" refers to a labeled oligonucleotide which forms a duplex structure with a sequence in the target nucleic acid, due to complementarity of at least a part of the sequence of the probe with a sequence in the target region. The probe is designated thus that it does not contain a sequence complementary to sequence(s) used to prime the polymerase chain reaction, or at least such a complementary sequence must be so small to ensure that no competitive hybridization occurs. Generally the 3' terminus of the probe will be "blocked" to prohibit incorporation of the probe into a primer extension product. "Blocking" can be achieved by using non-complementary bases or by adding a chemical moiety such as biotin or a phosphate group to the 3' hydroxyl of the last nucleotide, which may, depending upon the selected moiety, serve a dual purpose by also acting as a label for subsequent detection or capture of the nucleic acid attached to the label. Blocking can also be achieved by removing the 3'-OH or by using a nucleotide that lacks a 3'-OH such as a dideoxynucleotide.

It is also possible, however not preferred, that the probe is incorporated into the strain synthezised by the polymerase. Such incorporation might be desirable if the primer and the probe each carry part of a labeling system that upon interaction shows an amendment of a detectable and quantifiable signal.

The term "label" comprises any detectable and quantifiable signal that can be attached to a nucleic acid or protein. Preferred labels provide directly detectable signals, e.g. signals detectable by fluorescence, radioactivity, colorimetry, magnetism, enzymatic activity, and the like.

Polymerases suitable for the present invention have 5' to 3' nuclease activity, are temperature stable up to 95°C and ensure that nucleotides are removed from the 5' end of the probe in a sequential manner. Such polymerase are known (see e.g. WO 92/02638). Alternatively also polymerases incorporating a probe can be used in the presence of a suitable labeling-system.

In order to optimize the rapidity of the method, the primer and probe are preferably separated by 1 to about 800 nucleotides, more preferably about 1 to 400 nucleotides, and most preferably about 1 to 20 nucleotides.

As used herein, the term "polymerase" refers to an enzyme which is relatively stable to heat and preferably hydrolyzing intervening annealed probe to release both labeled and unlabeled probe fragments, or possibly incorporating intervening annealed probe, until synthesis terminates. A presently preferred thermostable enzyme isolated from *Thermus aquaticus (Taq)* is described in U.S. Patent No. 4,889,818 and a method for using it in conventional PCR is described in Saiki et al., 1998. Taq DNA polymerase has the needed exonuclease activity (see Gelfand, 1989).

According to the method of the present invention, and for an mRNA target, first transcription to cDNA is performed at temperatures of from 32 to 50°C, preferably from 37 to 48°C. The amplification/quantification reaction is then performed in cycles. First the strands are separated at temperatures of from about 85 to about 96°C, preferably from 90 to 95°C. Upon cooling to the annealing temperature of the primer, preferably about 57 to 61°C, due to the difference in the melting temperatures, first the probe is annealed and then the primers. As soon as the primers are annealed the polymerase starts synthesis of the complementary strand starting from the 3'-end of the primer towards the probe of which, in a preferred embodiment, mononucleotides or small oligonucleotides are cleaved by the 5'-3' nuclease activity of the polymerase thereby influencing the label system of the probe so that uncleaved labeled probe can be distinguished from the cleaved fragments thereof.

In order to get single strands, denaturation usually is performed at 85 to 95°C thus requiring temperature stable polymerases as e.g. described in WO 92/02638 and EP 632 134.

The separated nucleic acid strands are then incubated with primers and probes (preferably selected according to the present invention) under the above outlined hybridization conditions.

In order to avoid problems due to double-strand formation of the target with its complementary strand, a high molar excess of probe(s) and primers, is used.

As already mentioned above, the primers preferably have a hybridizing length of 18 to 22 bp. This allows for up to two mismatches provided that the last six nucleotides at the 3' end have a perfect match. A non hybridizing 5'-end does not influence the inventive method, whereby a non hybridizing sequence at the 3' end has to be avoided.

With regard to the probe, it preferably has a hybridizing length of 24 to 30 bp, whereby shorter probes may be used to distinguish possible targets and longer probes to allow more mismatches, and whereby additional non hybridizing bp are not critical and can even be advantageous, especially at the 5'-end with regard to a faciliated cleavage of a 5' situated label.

To ensure that the probe has sufficiant time to anneal, the probe can be situated so that the 5'-end of the probe is relatively far from the 3'-end of the primer, thereby giving the probe more time to anneal before primer extension blocks the probe binding site.

In order to enhance the Tₘ or thermal stability of the probe; for example, the nucleotide composition of the probe can be chosen to have greater G/C content. This can of course also be used to enhance Tₘ or thermal stability, respectively, of the primers. In similar fashion, one can incorporate modified nucleotides into the probe (primers), which modified nucleotides contain base analogs that form e.g. more stable base pairs than the bases that are typically present in naturally occurring nucleic acids.

In order to improve annealing of the probe, e.g. by securing the melting temperature difference, positively charged or neutral phosphodiester linkages can be incorporated in the probe to decrease the repulsion of the polyanionic backbones of the probe and target. Other methods are: the incorporation of alkylated or halogenated bases, such as 5-bromouridine, in the probe to increase base stacking; the incorporation of ribonucleotides into the probe to force the probe:target duplex into an "A" structure, which has increased base stacking; and the substitution of 2,6-diaminopurine (amino adenosine) for some or all of the adenosines in the probe (see WO 92/02638).

A general transcription process with following heating/cooling cycles of the method of the present invention comprises the following temperatures, heating/cooling steps and holding times:

To additionally favor binding of the probe before the primer, a molar excess of probe to primer concentration could be used, however, it was found that the present method even works with an excess of primer (about 4 molecules of primer to one molecule of probe).

The primers and probes may be prepared by any suitable method for the preparation of oligonucleotides which are known in the art, and the probe can be labeled, as described in WO92/02658.

Presently preferred is the use of two interactive labels on a single oligonucleotide such as Rhodamine and crystal violet or 6-Carboxyfluorescein and 6-carboxytetramethylrhodamine. It is of course also possible to use a label system with one component placed on the primer and a second component placed on the probe inducing or quenching a signal upon interaction due to polymerase activity.

In another embodiment, two preferably differently labeled probes complementary to separate regions of the same strand are used in order to enhance security and reliability of the analyse.

In a further embodiment two probes are used, each complementary to separate regions of separate strands of a double-stranded target region, but not to each other, so that each of the two probes anneals downstream of each primer.

Of course two or more probes can be prepared to search for the same or different mutants of the same pathogen.

It is also within the scope of the present invention to use two or more probes to accumulate, or achieve additional, benefits. For instance, one could test for more than one pathogen (the term pathogen includes various mutants) in a sample simply by putting several probes specific for different pathogens into the reaction mixture; the probes preferably comprise a different label each to facilitate detection, e.g. fluorescent markes emitting at different wave length and respective quenchers.

Suitable DNA polymerases include, for example, *Thermus flavus (Tfl)* DNA polymerase, *Thermus thermophilus (Tth)* DNA polymerase, *Bacillus stearothermophilus* DNA polymerase, *Thermococcus litoralis* DNA polymerase and the presently preferred *Thermus aquaticus (Taq)* DNA polymerase. The reaction conditions for catalyzing DNA synthesis with these DNA polymerases are well known in the art, whereby it has to be secured that the polymerizing agent efficiently cleaves the probe and releases labeled fragments so that the signal is directly or indirectly generated.

As in known processes (e.g. WO 92/02638) the reaction mixture - preferably after reverse transcription - is cycled through a denaturing step, a probe and primer annealing step, and a synthesis step, whereby cleavage and displacement occurs simultaneously with primer-dependent template extension. A DNA thermal cycler, such as the commercially available machine from Perkin-Elmer ABI Prism 7700 Sequence Detection System, which is specifically designed for use with a thermostable enzyme, may be employed.

As already mentioned, temperature stable polymerases are important in the present method, in particular in an automated process, because the preferred way of denaturing the double stranded extension products is by exposing them to a high temperature (about 95°C) during the PCR cycle. A suitable thermostable enzyme isolated from *Thermus aquaticus* is e.g. disclosed in U.S. Patent No. 4,889,818. Further temperature stable polymerases include polymerases extracted from the thermostable bacteria *Thermus flavus, Thermus ruber, Thermus thermophilus, Bacillus stearothermophilus, Thermus lacteus, Thermus rubens, Thermotoga maritima, Thermococcus litoralis,* and *Methanothermus fervidus.*

Efficacy of thermo stable polymerases is increased if these enzymes are protected either by antibody or by other molecules resulting in the constant and slow release of active enzyme with every heating process. This goal is reached e.g. by use of TaqGold marketed, e.g. by Perkin-Elmer Inc. Use of such a heat protection system in addition allows the so-called hot start method which results in a decreased effect of non-specific binding which occurs if a cold start has to be carried out.

Reagents employed in the methods of the invention can be packed into diagnostic kits comprising the at least one probe and the at least one set of primers in separate containers. If desired, the probe(s) can be unlabeled, and specific labeling reagents may also be provided as separate component in the kit.

The method of the present invention is very broadly applicable. It is suitable to detect and quantitate e.g. viruses, bacteria, rickettsia, mRNAs coding for cytokines, chemokines, or other proteins, whereby it is of outstanding interest for the detection of infections by viruses since viruses are known to possibly mutate at high mutation rates. Especially for such fast mutating viruses, but also for viruses and bacteria with relatively stable genes, the method of the present invention is very favorable.

Another embodiment of the present invention provides the presence of a so called housekeeping gene as additional means for standardization of the quantification. Although such a housekeeping gene with the method of the present invention is likely to be superfluous, it nevertheless adds to the security of the obtained result.

The present invention is not only applicable to a broad variety of pathogenic organisms but also to a broad variety of possible host organisms. Such organisms include all animals, whereby of course higher animals such as mammals including man are preferred, but also plants.

The invention is now further described with regard to mammals, namely specific cat viruses.

### Examples

All probe and primer sequences in the following examples are given in 5'-3' orientation.

### Example 1

In this first example a fluorogenic probe-based one-tube FCoV RT-PCR assay based on the reverse transcription activity of *avian myeloblastosis virus* reverse transcriptase (*AMV*-RT) in combination with the polymerisation and 5' nuclease activity of *Thermus flavus (Tfl)* polymerase is described. For absolute quantitation, a dilution series of FCoV RNA-standard is amplified with the samples. The assay is based on the reverse transcription and amplification of a portion of the FCoV 7b gene, which is known to be highly conserved among coronavirus isolates (Herrewegh et al. 1995).

A fluorogenic probe (a 25 base target cDNA specific oligonucleotide which is added to the RT-PCR mixture) was designed according to the present invention to hybridise to a sequence located between the PCR primers (FIG 1). A fluorescent reporter dye FAM (6-carboxy-fluorescein) was covalently attached to the 5' end of the probe and a quencher dye TAMRA (6-carboxytetramethylrhodamine) was covalently attached to the 3' end of the probe. The quencher dye absorbs the emission of the reporter dye by Förster-type energy transfer while the probe is intact (Förster, 1948). The 3' end of the probe was phosphorylated to prevent extension by the *Tfl* during the polymerisation steps. While the reporter dye emission is quenched due to the physical proximity of the quencher dye in the intact probe, the 5'→3' exonuclease activity of *Tfl* polymerase leads to cleavage of the probe in the extension phase of the PCR cycle. The effect is a dislocation of the reporter dye and, as a result of the decrease of the energy transfer, a rise of the reporter's fluorescence. The resulting relative increase of the reporter dye's fluorescencent emission was measured in the course of every PCR cycle using a Perkin-Elmer ABI Prism 7700 Sequence Detection System, which is a combination of thermal cycler and luminescence spectrometer. The data were transferred to a connected computer where calculations and graphics are demonstrated by a special software.

### Example 1A: Primers and probe

Primers and probe sequences (see below) were designed with Primer Express software (Perkin-Elmer, Foster City, USA).

The FCoV primers and probe for the FCoV fluorogenic assay were as follows with the length of the whole amplicon (fragment) being 102 bp:

Forward primer FCoV1128f (SEQ ID No.1)
GAT TTG ATT TGG CAA TGC TAG ATT T
Revers primer FCoV1229r (SEQ ID No.2)
AAC AAT CAC TAG ATC CAG ACG TTA GCT
Probe FCoV1200p (SEQ ID No.3)
(i)TCC ATT GTT GGC TCG TCA TAG CGG A(ii)

(I) Reporter dye (FAM) labeled nucleotide
(ii) Quencher dye (TAMRA) labeled nucleotide

A first selection of possible probe sequences was made in conformity to the manufacturers guidelines (Livak et al., December 1995), which are briefly (a) a melting temperature of approximately 5°C above the annealing temperature, (b) no complementarity to forward or revers primer, (c) no self-complentarity, (d) not four or more identical nucleotides in a row, especially Gs, (e) no G at the 5' end of the probe and (f) a slightly increased melting temperature compared to that of the primers. The TaqMan™ fluorogenic probe of the finally selected probe was synthesised by PE, Weiterstadt, Germany. The fluorescent reporter dye at the 5' end of the FCoV probe was FAM (6-carboxyfluorescein) and the rhodamine quencher dye at the 3' end was TAMRA (6-carboxytetramethylrhodamine). A phosphate group was added at the 3' end of the probe to prevent extension during polymerisation.

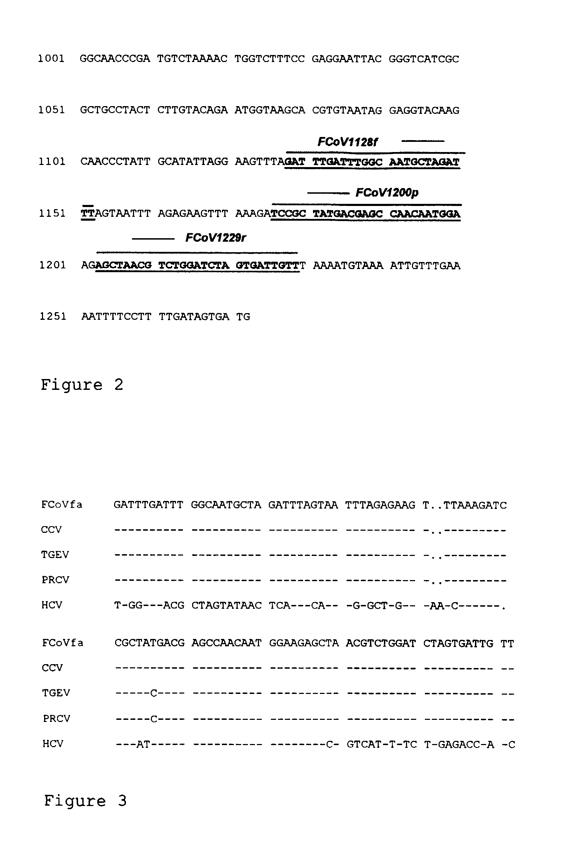

The primer pair for the FCoV fluorogenic probe was selected by paying attention that there was no loop or dimer formation with the other primers and a melting temperature of approximately 59°C. Because all FCoV strains should be detectable, probe and primers were designed according to the present invention to have a high crossreactivity with all known FCoV strains (for the location of the primers and the probe see Figure 2). A compilation of sequence comparisons and the crossreactivity of the inventive FCoV fluorogenic approach with different coronaviruses is shown in Figure 3. The Genetics Computer Group (GCG, University of Wisconsin) sequence analysis package was used for sequence analysis.

### Example 1B: FCoV standard for absolute quantitation

Standard RNA templates for FCoV were created as described elsewhere (Sambrook et al., 1989). Briefly, complementary DNA (cDNA) from an American FCoV strain was amplified by PCR with primers FCoV1128f and FCoV1229r. The fragment was separated on a 2 % agarose gel, excised, isolated from the gel and ligated into the polylinker of a pT7-blue vector (Novagen, Madison, WI, USA). The recombinant plasmid (named pT7-StFCoV) was transformed into *E.coli* (NovaBlue, Novagen, Madison, USA) and a positive colony was amplified in LB-medium (Luria-Bertani Medium) containing ampicillin. The plasmids were purified (Birnboim and Doly, 1979), linearised, purified by gel electrophoresis and extracted from the gel using a DEAE membrane (Sambrook et al., 1989). The standard plasmids were quantified by spectrophotometrical analysis. Verification and orientation of the sequence was verified by sequencing. The amount of 1.5µg of the linearised plasmid was used to perform an in vitro transcription with T7 polymerase (Boehringer Mannheim, Mannheim, Germany) at 37°C for 2 hours. After digestion with RNase free DNase, the resulting RNA transcripts were purified with phenolchloroform and precipitated with isopropanol. The RNA transcripts were disolved in diethylpyrocarbonate (DEPC) treated water and quantified by spectrophotometrical analysis. The dilutions of the standard plasmid were carried out in TE buffer (pH 7.6) containing 30µg carrier RNA (transfer RNA from *E.coli,* Sigma, Buchs, Switzerland) per ml. Endpoint dilution amplification was used to confirm the calculated copy number. The diluted RNA stock was aliquoted and frozen immediately at -70°C. Each aliquot was only used once for a fluorogenic RT-PCR.

### Example 1C: Fluorogenic RT-PCR

The 50 µl PCR mixture for one reaction contained 7µl optimized single-buffer 5x (Access RT-PCR system, Promega Corp., Madison, USA), 1.5µl PCR buffer lOx (500mM KCl, 100mM Tris-HCl (pH 8.3), passive reference dye ROX), 2 mM MgSO₄, 500 µM deoxynucleotide triphosphates, 0.1% Triton X-100 (t-octylphenoxypolyethoxyethanol, Sigma, Buchs, Switzerland), 1µm of each primer, 200nM of fluorogenic probe, 5U of AMV reverse transcriptase per reaction, 5U of *Tfl* polymerase per reaction and 10 µl of diluted standard or template RNA. After a reverse transcription step of 45 min at 48°C and a denaturation step of 2min at 94°C, 45 cycles, each 1min at 64°C, 2min at 68°C and 30s at 94°C, followed. Reverse transcription and amplification were carried out in a single tube in a ABI Prism 7700 Sequence Detection System (Perkin-Elmer, Applied Biosystems, Foster City, USA) without modifying or moving the samples between RT and PCR.

Although the above described example was performed with higher amounts, it has turned out that at least 100 µM deoxynucleotide triphosphates, at least 50 nM of fluorogenic probe, at least 1U of AMV reverse transcriptase per reaction, and at least 0.5U of Tfl polymerase per reaction are sufficient.

### Example 1D: FCoV strains and other coronaviruses

To evaluate the crossreactivity of this FCoV RT-PCR assay, several FCoV strains and isolates from coronaviruses of other species than the cat were tested with the result, shown in the Table 1 below.

**TABLE 1.**

| Crossreactivity of the FCoV fluorogenic assay with several FCoV strains and coronaviruses from other species | | |
|---|---|---|
| Isolate | Kind of sample | Result |
| Feline coronavirus strains (FCoV) | | |
| | | |
| FIPV 204859 | cell culture supernatant | detectable |
| FIPV UCD 1 VPII | cell culture supernatant | detectable |
| UCD 3 | cell culture supernatant | detectable |
| UCD 5 | cell culture supernatant | detectable |
| FIPV UCD 8 | ascites fluid | detectable |
| FeCV UCD 1 | feces suspension | detectable |
| FeCV RM | feces suspension | detectable |
| | | |
| Canine coronavirus (CCV) | cell culture supernatant | detectable |
| | | |
| Transmissible gastroenterits virus (TGEV) | cell culture supernatant | detectable |
| | | |
| Porcine respiratory coronavirus (PRCV) | cell culture supernatant | detectable |
| | | |
| Porcine epidemic diarrhea virus (PEDV) | | |
| | | |
| Isolate 1 | cell culture supernatant | not detectable |
| Isolate 2 | cell culture supernatant | not detectable |
| Isolate 3 | cell culture supernatant | not detectable |
| | | |
| Human coronavirus (HCV) strain 229E | | |
| | | |
| Isolate 1 | cell culture supernatant | not detectable |
| Isolate 2 | cell culture supernatant | not detectable |

Cell culture supernatants were used without being modified whereas feces samples and ascites fluid were diluted 1:10 and treated at 95°C for 5 minutes to eliminate RT-PCR inhibiting factors.

### Example 1E: RT-PCR data analysis

During amplification, fluorescence intensity of FAM, TAMRA and ROX in each tube of a 96 well plate was measured by the Perkin-Elmer ABI Prism 7700 Sequence Detection System. Fluorescence was monitored after each cycle when running the «real time» mode or only at the end point of PCR running the «plate read only» mode. The «real time» mode was accordingly used for quantitative PCR, the «plate read only» mode for qualitative results. Data were transferred to a connected computer and saved. Calculations and graphic presentation of the generation of fluorescence were carried out by the Sequence Detection System Software (SDS), which is part of the ABI Prism 7700 Sequence Detection system. For statistical analysis data were exported into a Microsoft Excel worksheet.

Running the «real time» mode, the SDS software offers two possibilities for data anlaysis, ΔRn and C_{T} (threshold cycle value) calculation. The ΔRn calculation mode is based on the Rₙ value, which is the ratio of the fluorescence signal between the reporter dye and the passive reference ROX. During PCR, Rₙ increases due to the cleavage of a probe with every copy of target that is produced, until the reaction reaches its plateau. ΔRₙ represents the normalised reporter signal minus the base line signal established in the first few cycles of PCR. Like Rₙ, ΔRₙ increases during PCR as amplicon copy number increases until the reaction approaches a plateau.

The threshold cycle (C_{T}) represents the PCR cycle at which an increase in reporter fluorescence above a base line signal can first be detected. A threshold of ten standard deviations above the base line is used to determine the C_{T} values. Because of a correlation between C_{T} value and starting copy number of the template, the C_{T} value is used for quantitation of the initial template number in the samples. The Sequence Detection software generates a standard curve of C_{T} vs starting copy number for all standards and then determines the starting copy number of unknowns by interpolation.

The specificity of the fluorogenic RT-PCR was confirmed by sequencing the linearised plasmid pT7-StFCoV by the chain termination method (Microsynth, Balgach, Switzerland). Two sequencing attempts showed right insert and right orientation of the insert in the plasmid.

### Results and Discussion

The analytical sensitivity of the inventive assay was 10 to 100 times better than the conventional nested RT-PCR (Herrewegh et al., 1995). A dilution series of a modified life FCoV vaccine (Primucell-FIP™, Pfizer animal health group, Belgium) was prepared and tested in triplicate by the fluorogenic RT-PCR according to the present invention and by nested RT-PCR. Nested RT-PCR products were evaluated by agarose gel electrophoresis. Whereas the conventional nested RT-PCR detected the FCoV down to a 1:10⁷ dilution (2 of 3 positive), the inventive method detected the whole virus even to a 1:10⁹ dilution (1 of 3 positive) (Table 2).

**TABLE 2.**

| Sensitivity of FCoV fluorogenic assay and nested FCoV RT-PCR | | | | | | |
|---|---|---|---|---|---|---|
| Dilution | Fluorogenic assay | | | Nested RT-PCR | | |
| 1/10⁴ | + | + | + | + | + | + |
| 1/10⁵ | + | + | + | + | + | + |
| 1/10⁶ | + | + | + | + | + | + |
| 1/10⁷ | + | + | + | + | + | - |
| 1/10⁸ | - | + | + | - | - | - |
| 1/10⁹ | + | - | - | - | - | - |
| 1/10¹⁰ | - | - | - | - | - | - |
| 1/10¹¹ | - | - | - | - | - | - |

To determine the reproducibility of the fluorogenic RT-PCR dilutions of the standard (50, 500 and 5000 molecules of standard RNA) and viral RNA (1:10⁶, 1:10⁵ and 1:10⁴ dilutions of a modified life FCoV vaccine) were prepared. The precision within-run was tested by pipetting the same dilutions 10 times on the same 96-well reaction plate, the precision from run-to-run by running the same dilutions in 10 different RT-PCR's. In all RT-PCR's all standard samples and samples with viral RNA were detected. The coefficient of variation (in percent) of the precision within run and from run to run was in the range of 0.57 to 1.50 and 1.16 to 3.42, respectively (Table 3).

**TABLE 3.**

| Coefficient of variation (in %) of precision within-run and precision from run-to-run | | | | | | |
|---|---|---|---|---|---|---|
| | standard RNA | | | complete virus | | |
| | 50 copies | 500 copies | 5000 copies | 1:10⁶ dilution | 1:10⁵ dilution | 1:10⁴ dilution |
| CV within-run | 1.50 | 0.88 | 0.69 | 0.57 | 0.91 | 0.59 |
| CV from run-to-run | 3.42 | 2.10 | 1.70 | 2.82 | 2.09 | 1.16 |

To assess the risk of crosscontamination, either by PCR products of previous reactions or during pipetting on the 96-well plate, 120 no template controls (containing water instead of the regular template) were included between the samples for reproducibility testing. To decrease the risk of crosscontamination, pipetting was performed in a laminar flow hood. The 96-well plates used in the 7700 ABI Prism system might generate a problem of crosscontamination because pipetting has to be done with the PCR tubes open. All the wells used are only closed with rows of linked caps after pipetting. However, none of the 120 no template controls gave a positive signal indicative for crosscontamination on the 96-well plate. The risk of crosscontamination can therefore be considered as very low, when pipetting is done in a laminar flow hood.

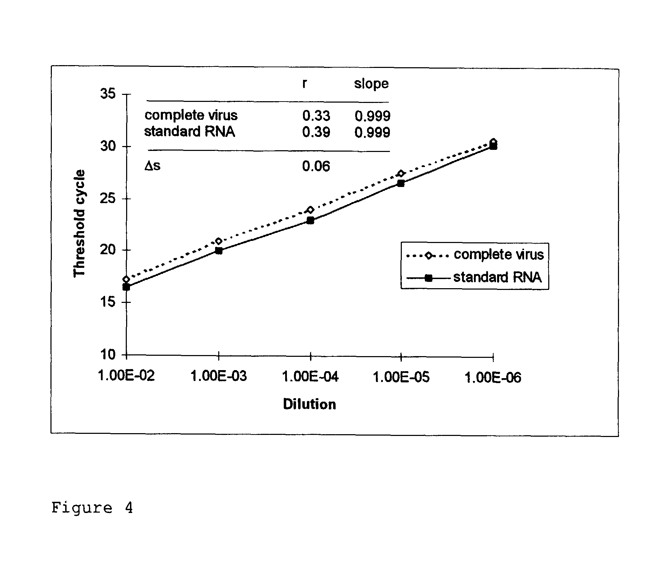

To verify that the amplification efficiency of the FCoV standard RNA transcripts is equal to that of the same sequence within the viral genome, the efficiencies of amplification of both templates has to be shown to be approximately equal. A very sensitive method to compare the efficiencies of amplification is to compare the slope of the standard curve of a standard RNA dilution series to the slope of the standard curve of a dilution series of complete virus particles. The efficiencies can be considered as equal if the difference of the slopes (As) is smaller than 0.1. In this approach, the As value was 0.06 (Figure 4), what proves that these standard RNA transcripts are reliable for FCoV qantification.

Several isolates were used to determine the crossreactivity of this RT-PCR assay. The expected results, based on the sequence comparisons, were confirmed: all tested FCoV isolates as well as CCV, TGEV and PRCV were detectable, HCV 229E and PEDV were not detectable (see Table 1).

PCR is an extremely sensitive method for the detection of specific nucleic acid sequences. A disadvantage of conventional PCR is that this method is a very time-consuming process, especially if the template is RNA and a reverse transcription step is needed or if a second round of amplification is necessary to achieve the required sensitivity. Furthermore, such steps increase the risk of inaccuracy and contamination. Several RT-PCR assays have been developed to detect FCoV (Gamble et al., 1997; Herrewegh et al., 1995; Li and Scott, 1994). Two of those assays are nested PCR's and in all of those ethidium bromide staining and UV light transillumination of electrophoretically separated PCR products is necessary to detect PCR products. A further big disadvantage of all those RT-PCR's is that none of them is able to quantitate FCoV.

The one-tube fluorogenic RT-PCR assay for FCoV quantitation described here is fast, simple and reliable. The base of this assay is a specific fluorogenic oligonucleotide synthesised according to the present invention to allow broad identification. Said oligonucleotide is provided with a labelling system known from the so-called TaqMan™ probe, which is added to the RT-PCR mix. This fluorogenic probe is cleaved during the PCR polymerisation steps due to a 5' nuclease activity of the Thermus flavus (Tfl) polymerase. While the unhybridised probe does not contribute significantly to the signal, the cleavage of the probe leads to the generation of fluorescence. This fluorescence is measured during each cycle by a luminescence spectrometer (ABI Prism 7700, Perkin-Elmer, Foster City, USA) and transferred to a connected computer, where graphics and calculations, for example standard curves or initial amounts of template in the samples, are automatically performed. The use of such a fluorogenic probe increases the level of specificity of the assay. This assay is a one-tube RT-PCR, where all ingredients for RT and PCR are mixed and RT-PCR is performed without additional handling or reopening of the tubes. No post amplification steps are required and calculation of the initial amount of viral particles is done by the software, resulting in a very short processing and assay time. RT-PCR testing of about 90 samples, including pipetting, can be performed on a 96 well plate including some non template controls and standard dilutions in less than 6 hours. The standard described, a cloned part of the highly conserved FCoV 7b gene, allows absolute quantitation of FCoV in clinical samples.

The precision within run and from run to run was evaluated with dilutions of the standard RNA transcripts and dilutions of a modified life vaccine (Primucell-FIP™, Pfizer animal health group, Belgium). The coefficient of variation of the precision within run and from run to run was in the range of 0.57% to 1.50% and 1.16% to 3.42%, respectively (see Table 3). Thus, the reproducibility in this assay is better than in conventional RT-PCR, where, for example, the precision within run is in the range of 2-7% (Kinoshita et al., 1992; Nagano and Kelly, 1994; Wang et al., 1989) .

The analytical sensitivity of the assay described here was 10 to 100 times higher than that of the conventional nested PCR (see Table 2). This sensitivity combined with the capability of quantitation and the specific security to specifically get the desired target(s) makes th is assay a valuable tool for FCoV research and diagnosis of FCoV infection.

The risk of contamination has been shown to be very low when pipetting is done in a laminar flow hood and when the different working steps were strictly separated. The fact, that the RT-PCR tubes are never opened after the PCR process, is an advantage of the fluorogenic RT-PCR, that reduces the risk of contamination as well.

The standard template was shown to be useful for quantitation of the complete viral RNA, based on the amplification efficieny which was shown to be approximately equal for both amplicons.

This FCoV assay was designed to detect all strains of FCoV. It is also useful for the detection of other coronaviruses, such as canine coronavirus (CCV), transmissible gastroenteritis virus (TGEV) and porcine respiratory coronavirus (PRCV) (see Table 1). The FCoV sequence selected in the present study has, as desired, a high conformity to CCV, TGEV and PRCV but only a low conformity to HCV 229E. All FCoV strains tested and several coronaviruses of other species than the cat were detected by the assay revealing its wide crossreactivity. HCV 229E and PEDV were in fact not detectable PEDV presumably since it is known to lack the 7 b gene.

These results show that the inventive one-tube fluorogenic RT-PCR for FCoV quantitation allows absolute quantitation, is highly sensitive, reliable, fast, easy to handle, enables a high sample throughput and contains a low risk of contamination. These characteristics make this assay an excellent tool for the detection of FCoV.

### Example 2

Feline immunodeficiency virus (FIV), a lentivirus first isolated in California (USA) by Pedersen and coworkers (1987), is morphologically and genetically similar to the human immunodeficiency virus (HIV) (Gardner et al., 1991). Because FIV is a naturally occurring infection inducing an AIDS-like disease in cats, FIV infection is considered to be an important animal model for the study of AIDS in human beings. Furthermore, the FIV model has proven to be useful for studying AIDS pathogenesis, evaluating new antilentiviral drugs, and establishing criteria for the development of safe and efficacious vaccines against lentiviral infections (Johnson et al., 1995; Bachmann et al., 1997; Leutenegger et al., 1998).

To study the effect of candidate vaccines or therapeutics, test systems are needed to quantify the FIV load for vaccine and therapy studies. Several PCR based methods have been proposed to quantify FIV provirus and viral RNA (Tomonaga and Mikami, 1996; Vahlenkamp et al., 1995; Diehl et al., 1995; Pistello et al., 1994; Cammarota et al., 1996, Allenspach et al., 1996). One of the most common concepts of quantitative competitive PCR (qcPCR) is the coamplification of different templates of similar lengths and with the same primer recognition sequences in the same tube, thus ensuring optimised thermodynamics and amplification efficiencies for both template species (Menzo et al., 1992; Siebert and Larrick, 1992) Usually several dilutions of the competitor DNA are coamplified with the same amount of genomic DNA (or RNA in the case of RT-PCR). After amplification, products of both template species can be detected by different methods: ELOSA (Enzyme Linked Oligonucleotide Sorbent Assay) combines a probe hybridisation technique with a simple method of detecting and quantifying PCR products in a microtiter format (Adler et al., 1992). Southern hybridisation is sensitive (Southern, 1975) but requires multiple post-PCR steps and can take several days before quantitation is completed. In gel electrophoresis, the two targets must be clearly distinguishable to allow densitometric evaluation of the relative intensities of the bonds. Because the templates compete for amplification (and in the case of competitive RT-PCR, also for reverse transcription), any variable affecting amplification has the same effect on both. Around the equivalence point, the ratio of PCR products reflects the ratio between the initial amount of the two sequences allowing precise evaluation of the amount of wild-type template. Another approach is the end-point dilution PCR, a method that was among the first developed (Simmonds et al., 1990; Zhang et al., 1993). However, the described PCR approaches have several important drawbacks (Clementi et al., 1993): (i) In particular, the techniques require several amplification reactions per sample, thus, they are expensive and allow the analysis of only a few samples for each amplification run. (ii) The postamplification steps are time consuming and may lead to inaccuracy and contamination or carryover. (iii) Quantitation using end-point dilution assay narrows the linear range to three to four log decades (Sundfors and Collan 1996). In conclusion, the conventional approach of qcPCR should be refined and improved by targeting the following criteria: (i) increasing sample throughput by eliminating parallel reactions for the quantitation of one sample, (ii) decreasing hands-on time by elimination of the postamplification steps, (iii) decreasing PCR costs, (iv) increasing reliability by enlarging the linear range of the PCR reaction.

The present example shows a much easier method for FIV detection based on a quantitative FIV PCR, namely the inventive real time PCR method. The method measures PCR product accumulation through a dual-labeled fluorogenic probe (i.e. the labelling system known for use in TaqMan probes; TaqMan, Perkin-Elmer Corp./Applied Biosystems, Foster City, California; Holland et al., 1991), taking advantage of the endogenous 5'-3' nuclease activity of *Taq* DNA polymerase to digest the probe which hybridizes to the amplicon during PCR. (For further details see Example 1.) Quantitation was accomplished by comparison of fluorescence signals obtained from samples with unknown FIV DNA load with the fluorescence signals obtained from FIV plasmid standard dilutions. Two probes were designed to detect different FIV isolates. Both probes were based on the PCR amplification of a portion of the FIV *gag* gene.

### Example 2A: Oligonucleotide probes and primers

Probe and primer sequences (see below) were designed with Primer Express software (Version 1, Perkin-Elmer, Foster City, California).

### A first primer/probe system designated

FIV0771f, FIV1081r and FIV1010p was obtained by simply following the joice of the computer system.

Forward primer FIV0771f (SEQ ID No 4)
AGAACCTGGTGATATACCAGAGAC

Reverse primer FIV1081r (SEQ ID No 5)
TTGGGTCAAGTGCTACATATTG
Probe FIV1010p (SEQ ID No 6)
(i)TATGCCTGTGGAGGGCCTTCCT(ii))

The whole length of the amplicon obtained by this method was 311 bp.

A further primer/probe system was chosen by the method of the invention. The inventive system is designated as FIV551f, FIV671r and FIV581p. 
Forward primer FIV551f (ID No 7)
GCCTTCTCTGCAAATTTAACACCT
Reverse primer FIV671r (ID No 8)
GATCATATTCTGCTGTCAATTGCTTT
Probe FIV581p (ID No 9)
(i)TGCGGCCATTATTAATGTGGCCATG(ii)
 (For the meaning of ⁽ⁱ⁾ and ⁽ⁱⁱ⁾ see above, Example 1)

The length of this amplicon was only 91 bp.

The probe sequence of the first system was selected based on the criteria according to Livak et al. 1995, namely melting temperature of the probe of 69°C, lack of predicted dimer formation with corresponding primers, lack of self-annealing, no stretches of identical nucleotides longer than four, no G at 5' end of probe, 10°C higher melting temperature of the probe than that of the primers. The probe sequence of the probe of the second system was selected according to the criteria of Livak et al., 1995 extended by the criteria of the present invention. The fluorescent reporter dye at the 5 ends of both FIV probes was FAM (6-carboxyfluorescein). From the sequences obtained by applying these criteria, such ones meeting the requirements of the present invention were selected.

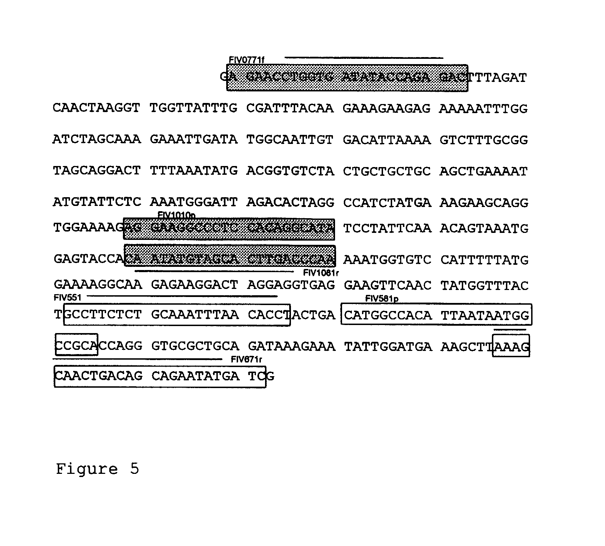

Such probes and primers were designed to have a high crossreactivity between *gag* sequences from different FIV subtypes. The location of the probes and primers is shown in Figure 5. Probe FIV581p was designed to be 100% homologous to FIV Zurich 2 (FIV Z2) and FIV Petaluma (FIV Pet). Probe FIV1010p was designed to be 100% identical to FIV Utrecht, Zurich 1, Amsterdam AM6, and San Diego, respectively. A compilation of sequence comparisons between the two probes and different FIV subtypes is given in Table 4.

**Table 4.**

| FIV *gag*-specific sequence similarities of probes with different FIV subtypes | | | | | |
|---|---|---|---|---|---|
| Subtype | Description | Match with probe (%) | | Numbers of mismatches (bp) | |
| | | FIV1010p | FIV581p | FIV1010p | FIV581p |
| A | FIV Petaluma (California) | 100 | 100 | 0 | 0 |
| | FIV Zurich 2 (Switzerland) | 95.5 | 100 | 1 | 0 |
| | FIV San Diego (USA) | 100 | 100 | 0 | 0 |
| | FIV Utrecht (NL) | 100 | 96 | 0 | 1 |
| B | FIV Sendai 2 (Japan) | 91 | 84 | 2 | 4 |
| | FIV Aomori 1/2 (Japan) | 86.5 | 84 | 3 | 4 |
| | FIV Tokyo 1 (Japan) | 86.5 | 84 | 3 | 4 |
| D | FIV Shizuoka (Japan) | 77 | 80 | 5 | 5 |
| | FIV Fukuoka (Japan) | 73 | 80 | 6 | 5 |

Sequences were analysed with the Genetics Computer Group (GCG, University of Wisconsin) sequence analysis package.

### Example 2B: DNA samples and FIV subtypes

Genomic DNA was prepared from peripheral blood mononuclear cells (PBMC) purified by Ficoll Hypaque gradient centrifugation from heparin treated blood samples. DNA extraction was performed with a phenolchloroform standard method (Chomczynski and Sacchi, 1987). Genomic DNA (500 ng) was used as template for amplification in the TaqMan PCR. Purified cat DNA harbouring different FIV subtypes were obtained from Japan (subtypes A, B and D, obtained by A. Hasegawa), from Europe (subtypes A and B, obtained by M. Horzinek, H. Egberink, A.D.M.E. Osterhaus, M. Bendinelli, and O. Jarrett), and from the United States (obtained by N. Pedersen).

### Example 2C: Plasmid standard for quantitation

A plasmid standard for FIV DNA PCR was prepared as described earlier (Allenspach et al., 1996). Briefly, genomic DNA (gDNA) from an FIV Pet infected cell line (FL-4, kindly provided by. J. Yamamoto) was amplified by PCR with primers within the *gag* gene showing high sequence homology between FIV Z2 and FIV Pet (both subtype A, Kakinuma et al., 1995). The 602 bp fragment was cloned into a Bluescript II plasmid (pBSII; Stratagene, La Jolla, CA, USA) and propagated in *E.coli* (BL21 pLysS, Novagen, Madison, WI, USA). Dilutions of linearized and purified plasmid standard contained 30 µg calf thymus DNA (Sigma, Buchs, Switzerland) per ml as a carrier. Aliquots of dilutions were frozen at -20°C and used only once in TaqMan PCR.

### Example 2D: Fluorogenic PCR

The 50 µl PCR mixtures contained 10 mM Tris (pH 8.3), 50 mM KCl, 75 mM passive reference dye ROX, 5 mM MgCl₂, 200 µM deoxynucleotide triphosphates, 500 nM of each primer, 100 nM of fluorogenic probe, 0.25 U of *Taq* DNA polymerase per reaction and 10 µl of diluted template or plasmid standard. After target denaturation for 3 min at 95°C, amplification conditions were: 5 cycles of 30 sec at 95°C, 30 sec at 64°C, followed by 40 cycles of 30 sec at 85°C, 60 sec at 64°C. Amplification, data acquisition and data analysis was carried out in an ABI Prism 7700 Sequence Detection System (Perkin-Elmer, Applied Biosystems, Foster City, California). Data were analysed with a Sequence Detection System Software (SDS, Version 1.6) and C_{T} values were exported into a Microsoft Excel worksheet for further statistical analysis. The specificity of the TaqMan PCR was confirmed by sequencing a PCR product.

### Seminested FIV PCR: The protocol for the seminested FIV PCR was described previously (Leutenegger et al., 1998)

*Determination of precision:* Precision within-run was calculated from testing 10 aliquots each of 50, 500 and 5000 plasmid standard molecules and an FIV Z2 positive gDNA sample in parallel at the same time. Precision between runs was calculated after testing of 50, 500 and 5000 copies of the FIV plasmid standard and an FIV Z2 positive genomic DNA sample on 10 different occasions.

### Results and Discussion:

*Analytical sensitivity:* To compare the analytical sensitivity of the seminested FIV PCR with the TaqMan PCR, end-point dilution amplifications were carried out with the same dilutions in both systems. The analytical sensitivity of the conventional seminested PCR (Leutenegger et al., 1998) was comparable to that achieved by the TaqMan PCR assay. Dilution series containing 0, 0.1, 1, 5, 10, 50, 100 and 500 molecules of plasmid standards was tested in triplicate. Seminested PCR products were evaluated by agarose gel electrophoresis. Whereas the conventional seminested PCR detected two out of three samples containing 1 molecule of plasmid standard, only one was scored positive in the TaqMan PCR. This result could be explained by the probability that 1 molecule may be present or absent in a volume of 10 microliter. All nontemplate controls and all samples containing 0.1 plasmid standard gave negative results in both systems. All other reactions scored positive in both tests.

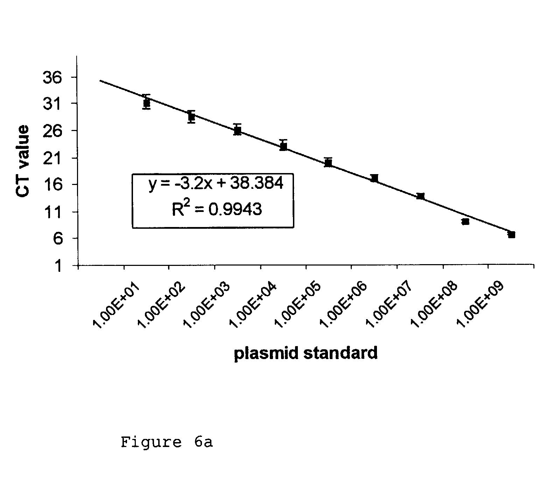

*Linearity:* Amplification of a plasmid standard dilution over 8 orders of magnitude showed linearity over the whole range (Figure 6a). To obtain linearity even at highest plasmid dilutions, a constant amount of carrier DNA was used to prevent adsorbing of plasmids to the tube walls.

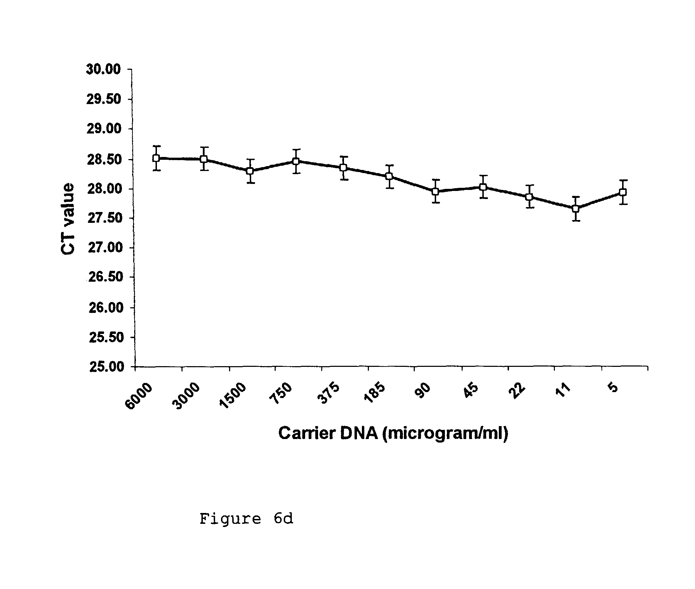

*Effect of carrier DNA* / Range *(Figure 6d):* To test the influence of the carrier DNA on the recognition of the plasmid standard in the TaqMan PCR, the plasmid standard was amplified against a broad range of carrier DNA. As the influence of the carrier DNA on large plasmid standard numbers may be expected to be smaller, a low number of plasmid standard were used. Two types of carrier DNA were used: calf thymus DNA and gDNA from a FIV negative cat. 50 molecules of plasmid standards were amplified with varying amounts of carrier DNA (calf thymus DNA, Sigma, Buchs, Switzerland; gDNA from an FIV negative cat) ranging from 6000 µg/ml to 5 µg/ml in a 50 µl reaction volume. 50 molecules could be detected throughout the whole range with calf thymus DNA (Figure 6d) with an increase in the C_{T} value of 0.5 at the highest concentration of carrier DNA (6000 µg/ml). A C_{T} value of 0.5 corresponds to a difference of 50% of target recognition. As a control, carrier DNA alone (and at highest concentration) gave negative results in the PCR method of the present invention. However, this slightly decreased analytical sensitivity is irrelevant as long as the same amount of carrier DNA is used in samples that are compared. All subsequent experiments were carried out with 30 µg/ml of carrier DNA. Calf thymus DNA and FIV negative gDNA from an uninfected cat as carrier DNA gave comparable results.

*Precision determination:* To assess the precision within-run and between-runs, dilutions of the plasmid standard (50, 500 and 5000 molecules of plasmid standards) and FIV Z2 positive genomic DNA were evaluated sample by using TaqMan PCR on 10 separate occasions. All the plasmid standard samples and samples with genomic DNA were positive each time with both probes FIV1010p and FIV581p. Coefficients of variation (CV) were calculated once on the basis of the C_{T} values (CV(C_{T})) and once with the absolute values of the initial copy numbers (CV(abs)). The CV(C_{T}) of the within-run precision was in the range of 0.29 to 1.6%, the CV(abs) in the range of 4.4 to 27.13%. The CV(C_{T}) of the between-run experiment was in the range of 0.88 to 2.27%, the CV(abs) in the range of 18.26 to 28.89% (Table 5).

**Table 5.**

| CV ( in %) of within-run and between-run precision. CV's were calculated on the basis of the CT values (CV(C_{T})) and with the absolute values of the initial copy numbers (CV(abs)) | | | | | | | | |
|---|---|---|---|---|---|---|---|---|
| | probe | | | | | | | |
| | FIV1010p (comparision) | | | | FIV581p (invention) | | | |
| copy number/DNA: | 50 | 500 | 5000 | gDNA | 50 | 500 | 5000 | gDNA |
| CV(C_{T}) | | | | | | | | |
| within run | 1.13 | 0.96 | 0.29 | 0.43 | 1.26 | 1.0 | 0.96 | 1.6 |
| between run | 1.33 | 1.44 | 1.59 | 1.24 | 1.19 | 1.86 | 2.27 | 1.88 |
| | | | | | | | | |
| CV (abs) | | | | | | | | |
| within run | 24.1 | 17.7 | 4.4 | - | 27.13 | 18.51 | 15.28 | - |
| between run | 28.89 | 27.55 | 26.7 | - | 25.37 | 18.26 | 18.8 | - |

In general, both the within-run and between-run results showed better precision with higher numbers of standard plasmids.

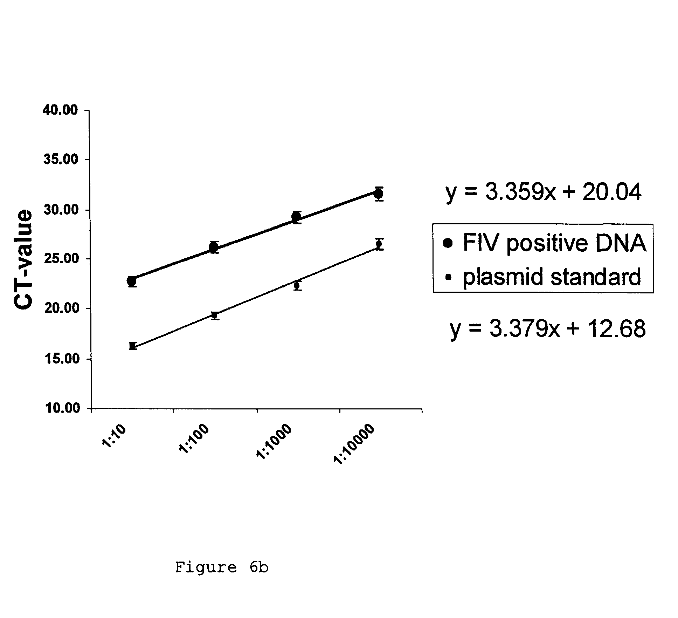

*Amplification efficiency* was determined with dilution series of plasmid standard and DNA. The standard curves for the plasmid standard and for gDNA were used to provide the necessary data. Figure 6b shows the average CT values for the plasmid standard and target gDNA. If the efficiencies of the plasmid standard and the gDNA are approximately the same, the difference of the slopes (Δs) of the two dilution curves should be smaller than 0.1. Efficiency determination for the plasmid standard and the gDNA revealed a Δs of 0.02 (3.379 vs 3.359).

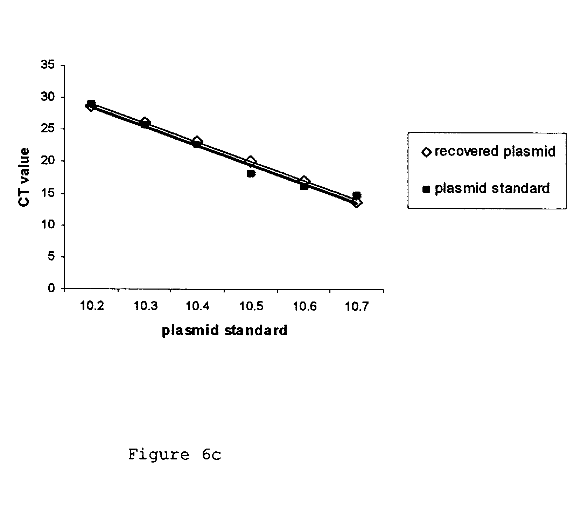

*Recovery experiment:* In order to evaluate the effects of DNA purification on template recovery and to further control the reliability of quantitative results, 5x10⁶ Ficoll purified feline PBMCs spiked with a series of plasmid standard (10², 10³, 10⁴, 10⁵, 10⁶ and 10⁷ standard molecules) were extracted and tested against a plasmid standard series in six repetitions (Figure 6c). A mean loss of 37% was observed. The r² of the recovered plasmid standard and directly analyzed plasmid standard were 0.992 and 0.976, respectively. The difference between the two slopes was 0.017.

*Risk of contamination and carryover:* The risk of contamination was assessed in parallel to the test for within-run precision. Between all plasmid standard or gDNA samples tested during the evaluation phase, a total of 480 no template controls (NTC) were included. When pipetting was done in a laminar hood, all 480 NTC remained negative indicative of no contamination on the 96 well plate. In contrast, when pipetting was carried out on a work bench, 2 out of 24 NTC showed positive results after amplification.

*Crossreactivity of probes and primers:* DNA from 33 different and well characterized FIV isolates were tested to evaluate the crossreactivity of the two probe-primer combinations. FIV isolates belong to the clades A, B and D according to a classification published by Kakinuma and coworkers (1995). According to published sequences, the degree of mismatch could be determined (Table 4). Most of the subtype A and B isolates could be detected with both probe-primer combinations (see Table 6 next page). With probe FIV581p, 18 of 20 subtypes A, 10 of 12 subtypes B, and 0 of 5 subtypes D, with probe FIV1010p 13/20 subtypes A, 10/12 subtypes B and 0/5 subtypes D could be detected (Table 6). This Example shows that the probe according to the invention is able to identify isolates and mutants leading to the considered amount of mismatches.

**Table 6.**

| Detection of different FIV isolates with the two probes FIV581p and FIV1010p | | | | | |
|---|---|---|---|---|---|
| | | probe | | | |
| | | FIV581p (inventive) | | FIV1010p (comparision) | |
| Isolate | subtype | detectable? | | detectable? | |
| FIV Z2 | A | yes | | yes | |
| FIV Pet (FL-4) | A | yes | | yes | |
| FIV Pet (CrFK) | A | yes | | yes | |
| Glasgow 1 | A | yes | | yes | |
| Glasgow 2 | A | yes | | yes | |
| Glasgow 5 | A | yes | | yes | |
| Glasgow 8 | A | yes | | yes | |
| Glasgow 14 | A | yes | | yes | |
| Utrecht 113 | A | yes | | yes | |
| Amsterdam 6 | A | yes | | yes | |
| Dutch F2663E | A | yes | | yes | |
| Dutch F2494E | A | no | | no | |
| Dutch F3045E | A | yes | | yes | |
| Dutch F3109E | A | no | | no | |
| Dutch F3120E | A | yes | | no | |
| Dutch F3046E | A | yes | | no | |
| Dutch F3127E | A | yes | | no | |
| Dutch F3432E | A | yes | | no | |
| Dutch F3144E | A | yes | | yes | |
| Japan 8 | A | yes | | no | |
| Italy M2 | B | yes | | yes | |
| Italy M8 | B | yes | | yes | |
| Italy M9 | B | yes | | yes | |
| Italy M14 | B | yes | | yes | |
| Italy M16 | B | yes | | no | |
| Italy M18 | B | yes | | yes | |
| Italy M20 | B | yes | | yes | |
| Italy M88 | B | yes | | yes | |
| Italy L99 | B | yes | | yes | |
| Italy L155 | B | yes | | yes | |
| Japan 6 | B | no | | no | |
| Japan 7 | B | no | | yes | |
| Japan 1 | D | no | | no | |
| Japan 2 | D | no | | no | |
| Japan 3 | D | no | | no | |
| Japan 4 | D | no | | no | |
| Japan 5 | D | no | | no | |
| Detection frequency: | | subtypes A | 18/20 | subtypes A | 13/20 |
| | | | | | |
| | | subtypes B | 10/12 | subtypes B | 10/12 |
| | | subtypes D | 0/5 | subtypes D | 0/5 |

The inventive FIV fluorogenic assay described here is an improvement on hitherto used methods based on the use of end-point dilution analysis or on the coamplification of a competitive internal standard together with the viral sequences. Such tests need parallel reactions of plasmid standard dilutions and timeconsuming postamplification steps. The inventive assay is a fast, simple and reliable alternative to quantify FIV provirus in genomic DNA from various tissues and feline PBMC's. It is a convenient, relatively inexpensive assay and is ideal to quantify large numbers of specimens. The elimination of the postamplification steps increases reliability and reproducibility of the assay, demonstrated by the high precision. Furthermore, loss of postamplification steps help to eliminate the risk of carryover. Importantly, amplification can be done in the absence of any competition or saturation phenomena. A total of e.g. 88 DNA samples can be quantified on one 96 well plate together with three non template controls and 5 dilutions of the plasmid standard. Thus, this methodology offers a significant saving of time: pipetting, amplification and quantitation of 88 samples to be quantified was achieved in less than 4 hours with hands-on-time of around 60 minutes.

In FIV research, high precision for the determination of provirus load is especially useful. Since it is the prerequisite to determine significant differences of virus load in groups of cats, e.g. vaccinated with different vaccines or treated with different antiretroviral drugs, high precision reduces the number of analysis required to obtain a significant result and therefore helps to reduce costs. Furthermore, and in contrast to conventional qcPCR protocols, the linear range is extended over 9 log decades. This prevents additional reactions in the case of a high provirus load.

To enable accurate quantitation by comparison of FIV DNA with plasmid standard, the efficiency of both amplifications should be approximately equal. A very sensitive method for assessing the amplification efficiency between two amplicons is to determine the C_{T} value variation from dilution series of plasmid standard and gDNA. These results showed, that the amplification efficiency of the plasmid standards and gDNA were comparable. Therefore, quantitation of the target gDNA can be carried out without limitation due to different amplification efficiencies. Furthermore, the recovery experiments showed that extraction of a series of plasmid standard dilutions from PBMC's does not lead to a changed amplification efficiency. Recovery of the standard plasmid dilutions revealed a certain loss of DNA during the extraction procedure. Absolute quantitation should therefore be corrected for this percentage loss determined specifically for the extraction method used. However, the absolute quantitative data presented in this report were not corrected to allow a direct approach to the PCR results obtained with the method of the present invention.

Care to reduce possible sources of contamination and carryover should be increased with the inventive method. Inclusion of the AmpErase® UNG system (Pang et al., 1992) may reduce the likelihood of false-positives due to carryover, whereby the loss of postamplification steps in any case eliminates the need for reducing the carryover problem. Nevertheless, good PCR practice like improved PCR reagents handling (aliquoting), pipetting in a laminar hood, strict separation of DNA containing samples and PCR reagents from amplified products has proved to help to prevent contamination and carryover. The results showed that the risk of contamination has to be considered as very low or even absent when pipetting was done in a hood ensuring laminar flow of the air.

To detect FIV subtypes in naturally infected cats, probe and primers should recognize a wide range of FIV subtypes. Inventive probe-primer combinations were therefore designed to be crossreactive with FIV subtypes from the A and B clade representing the majority of isolates in Europe. Testing of FIV isolates originating from different geographical areas and representing different subtypes has revealed that the two systems presented herein may detect a large variety of isolates. From these results two important conclusions have to be drawn: First, monitoring of naturally infected cats is possible with sufficient quantitation of the virus load. Only if very accurate quantitation of one particular FIV subtype has to be achieved, it might be preferable that probes and primers are designed which are 100% complementary to the target strain.

Overall, the results presented above show that application of the inventive method greatly simplifies the detection and quantitation of FIV provirus and allows increased sample throughput while retaining the exquisite sensitivity of conventional nested PCR. Furthermore, evaluation of precision and accuracy shows very good reliability of the inventive procedure for this measurement.

### Examples 3 to 27:

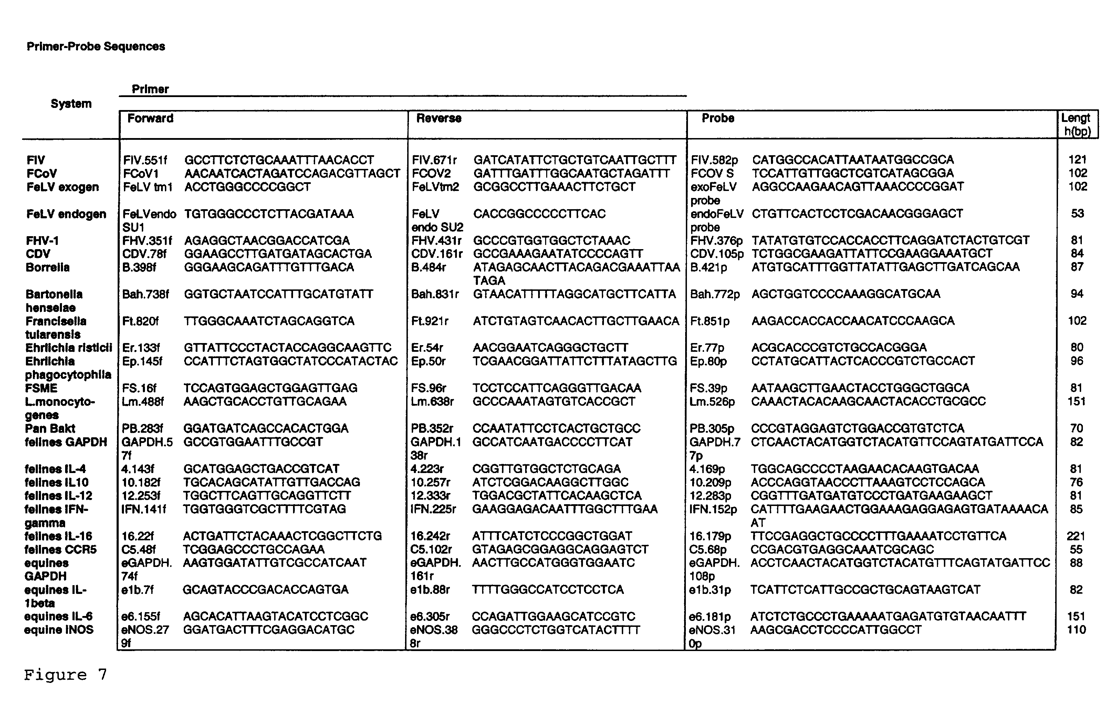

On the same bases further probe-primer-systems were developped and tested (see Table 7 at the end of the experimental part for an overal view of the sequences of Examples 3 to 26). Said probe-primer-systems are as follows, the length of the amplicon (in general between 100 and 225 bp) is shown in Figure 7:

### Example 3: FIV

Forward primer FIV.551f (SEQ ID No. 11, same as SEQ ID
No. 7)
GCCTTCTCTGCAAATTTAACACCT
Reverse primer FIV.671r (SEQ ID No. 10, same as SEQ ID
No. 8)
GATCATATTCTGCTGTCAATTGCTTT
Probe FIV.582p (SEQ ID No. 12 )
CATGGCCACATTAATAATGGCCGCA

Said system is especially suited for the simultaneous determination of several isolates.

### Example 4: FCoV

Forward primer FCoV1 (SEQ ID No. 13)
AACAATCACTAGATCCAGACGTTAGCT
Reverse primer FCOV2(SEQ ID No. 14)
GATTTGATTTGGCAATGCTAGATTT
Probe FCOV S(SEQ ID No. 15)
TCCATTGTTGGCTCGTCATAGCGGA

Said system is especially suited for the simultaneous determination of many isolates.

### Example 5: FeLV exogen

Forward primer FeLV tm1(SEQ ID No. 16)
ACCTGGCCCCGGCT
Reverse primer FeLVtm2(SEQ ID No. 17)
GCGGCCTTGAAACTTCTGCT
Probe exoFeLV probe(SEQ ID No 18)
AGGCCAAGAACAGTTAAACCCCGGAT

Said system is suitable for the simultaneous determination of subtypes A and B. In addition, said system recognizes some of the subtype C isolates of FeLV.

### Example 6: FeLV endogen

Forward primer FeLVendo SU1 (SEQ ID No. 19)
TGTGGGCCCTCTTACGATAAA
Reverse primer FeLV endo SU2(SEQ ID No. 20)
CACCGGCCCCCTTCAC
Probe endoFeLV probe(SEQ ID No 21)
CTGTTCACTCCTCGACAACGGGAGCT

Said system is especially suited for the selected determination of endogenous FeLV.

### Example 7: FHV-1

Forward primer FHV.351f(SEQ ID No. 22)
AGAGGCTAACGGACCATCGA
Reverse primer FHV.431r(SEQ ID No. 23)
GCCCGTGGTGGCTCTAAAC
Probe FHV.376p (SEQ ID No 24)
TATATGTGTCCACCACCTTCAGGATCTACTGTCGT

Said system is specific for the detection and quantification of the feline herpes virus (no interference with other herpes viruses).

### Example 8: CDV

Forward primer CDV.78f(SEQ ID No. 25)
GGAAGCCTTGATGATAGCACTGA
Reverse primer CDV.161r (SEQ ID No. 26)
GCCGAAAGAATATCCCCAGTT
Probe CDV.105p (SEQ ID No 27)
TCTGGCGAAGATTATTCCGAAGGAAATGCT

Said system is especially suited for the determination of the canine morbilli virus. Morbilli viruses of other species are generally not detected.

This assay is especially suited to be used as a control for RT(reverse transcriptase)-PCR applications in all species except canids.

### Example 9: Borrelia

Forward primer B.398f(SEQ ID No. 28)
GGGAAGCAGATTTGTTTGACA
Reverse primer B.484r (SEQ ID No. 29)
ATAGAGCAACTTACAGACGAAATTAATAGA
Probe B.421p (SEQ ID No 30)
ATGTGCATTTGGTTATATTGAGCTTGATCAGCAA

Said system is especially suited for the determination of the three Borrelia agents currently known to be important pathogens which are Borrelia burgdorferi sensu lato, Borrelia afzelii and garinii.

### Example 10: Bartonella henselae

Forward primer Bah.738f (SEQ ID No. 31)
GGTGCTAATCCATTTGCATGTATT
Reverse primer Bah.831r (SEQ ID No. 32)
GTAACATTTTTAGGCATGCTTCATTA
Probe B.772p (SEQ ID No 33)
AGCTGGTCCCCAAAGGCATGCAA

Said system is especially suited for the detection and quantification of B. henselae.

### Example 11: Francisella tularensis

Forward primer Ft.820f (SEQ ID No. 34)
TTGGGCAAATCTAGCAGGTCA
Reverse primer Ft.921r (SEQ ID No. 35)
ATCTGTAGTCAACACTTGCTTGAACA
Probe Ft.851p (SEQ ID No 36)
AAGACCACCACCAACATCCCAAGCA

Said system is especially suited for the detection and quantification of Francisella tularensis.

### Example 12: Ehrlichia risticii

Forward primer Er.133f (SEQ ID No. 37)
GTTATTCCCTACTACCAGGCAAGTTC
Reverse primer Er.54r (SEQ ID No. 38)
AACGGAATCAGGGCTGCTT
Probe Er.77p (SEQ ID No 39)
ACGCACCCGTCTGCCACGGGA

Said system is selective for isolates of the E. risticii genogroup.

### Example 13: Ehrlichia phagocytophila

Forward primer Ep.145f (SEQ ID No. 40)
CCATTTCTAGTGGCTATCCCATACTAC
Reverse primer Ep.50r (SEQ ID No. 41)
TCGAACGGATTATTCTTTATAGCTTG
Probe Ep.80p (SEQ ID No 42)
CCTATGCATTACTCACCCGTCTGCCACT

Said system is selective for the E. phagocytophilia geno group.

### Example 14: FSME (= CETE = Central European Tick Born Enzephalitis caused by flavi viruses in Central Europe, transmitted by ticks).

Forward primer FS.16f (SEQ ID No. 43)
TCCAGTGGAGCTGGAGTTGAG
Reverse primer FS.96r (SEQ ID No. 44)
TCCTCCATTCAGGGTTGACAA
Probe FS.39p (SEQ ID No 45)
AATAAGCTTGAACTACCTGGGCTGGCA

Said system is selective for a tick-borne flavi virus occurring in central Europe.

### Example 15: L. monocytogenes

Forward primer Lm.488f (SEQ ID No. 46)
AAGCTGCACCTGTTGCAGAA
Reverse primer Lm.638r (SEQ ID No. 47)

GCCCAAATAGTGTCACCGCT
Probe Lm.526p (SEQ ID No 48)
CAAACTACACAAGCAACTACACCTGCGCC

Said system is selective for the four serovars of L. monocytogenes.

In the following Examples systems for detection and quantification of feline cytokines and equine cytokines are described. Said systems only recognize the cytokine genes of the respective species.

### Example 16: Feline GAPDH

Forward primer GAPDH.57f (SEQ ID No. 49)
GCCGTGGAATTTGCCGT
Reverse primer GAPDH.138r (SEQ ID No. 50)
GCCATCAATGACCCCTTCAT
Probe GAPDH.77p (SEQ ID No 51)
CTCAACTACATGGTCTACATGTTCCAGTATGATTCCA

### Example 17: Feline IL-4

Forward primer 4.143f (SEQ ID No. 52)
GCATGGAGCTGACCGTCAT
Reverse primer 4.223r (SEQ ID No. 53)
CGGTTGTGGCTCTGCAGA
Probe 4.169p (SEQ ID No 54)
TGGCAGCCCCTAAGAACACAAGTGACAA

### Example 18: Feline IL-10

Forward primer 10.182f (SEQ ID No. 55)

TGCACAGCATATTGTTGACCAG
Reverse primer 10.257r (SEQ ID No. 56)
ATCTCGGACAAGGCTTGGC
Probe 10.209p (SEQ ID No 57)
ACCCAGGTAACCCTTAAAGTCCTCCAGCA

### Example 19: Feline IL-12

Forward primer 12.253f (SEQ ID No. 58)
TGGCTTCAGTTGCAGGTTCTT
Reverse primer 12.333r (SEQ ID No. 59)
TGGACGCTATTCACAAGCTCA
Probe 12.283p (SEQ ID No 60)
CGGTTTGATGATGTCCCTGATGAAGAAGCT

### Example 20: Feline IFN-gamma

Forward primer IFN.141f (SEQ ID No. 61)
TGGTGGGTCGCTTTTCGTAG
Reverse primer IFN.225r (SEQ ID No. 62)
GAAGGAGACAATTTGGCTTTGAA
Probe IFN.152p (SEQ ID No 63)
CATTTTGAAGAACTGGAAAGAGGAGAGTGATAAAACAAT

### Example 21: Feline IL-16

Forward primer 16.22f (SEQ ID No. 64)
ACTGATTCTACAAACTCGGCTTCTG
Reverse primer 16.242r (SEQ ID No. 65)
ATTTCATCTCCCGGCTGGAT
Probe 16.179p (SEQ ID No 66)
TTCCGAGGCTGCCCCTTTGAAAATCCTGTTCA

### Example 22: Feline CCR5

Forward primer C5.48f (SEQ ID No. 67)
TCGGAGCCCTGCCAGAA
Reverse primer C5.102r (SEQ ID No. 68)
GTAGAGCGGAGGCAGGAGTCT
Probe C5.68p (SEQ ID No 69)
CCGACGTGAGGCAAATCGCAGC

### Example 23: Equine GAPDH

Forward primer eGAPDH. 74f (SEQ ID No. 70)
AAGTGGATATTGTCGCCATCAAT
Reverse primer eGAPDH. 161r (SEQ ID No. 71)
AACTTGCCATGGGTGGAATC
Probe eGAPDH. 108p (SEQ ID No 72)
ACCTCAACTACATGGTCTACATGTTTCAGTATGATTCC

### Example 24: Equine IL-lbeta

Forward primer e1b.7f (SEQ ID No. 73)
GCAGTACCCGACACCAGTGA
Reverse primer e1b.88r (SEQ ID No. 74)
TTTTGGGCCATCCTCCTCA
Probe e1b.31p (SEQ ID No 75)
TCATTCTCATTGCCGCTGCAGTAAGTCAT

### Example 25: equine IL-6

Forward primer e6.155f (SEQ ID No. 76)
AGCACATTAAGTACATCCTCGGC
Reverse primer e6.305r (SEQ ID No. 77)
CCAGATTGGAAGCATCCGTC
Probe e6.181p (SEQ ID No 78)
ATCTCTGCCCTGAAAAATGAGATGTGTAACAATTT

### Example 26: Equine iNOS

Forward primer eNOS.279f (SEQ ID No. 79)
GGATGACTTTCGAGGACATGC
Reverse primer eNOS.388r (SEQ ID No. 80)
GGGCCCTCTGGTCATACTTTT
Probe eNOS.310p (SEQ ID No 81)
AAGCGACCTCCCCATTGGCCT

### Example 27: Pan Bakt

Forward primer (SEQ ID No. 82) PB.283f
GGATGATCAGCCACACTGGA
Reverse primer (SEQ ID No. 83) PB.352r
CCAATATTCCTCACTGCTGCC
Probe (SEQ ID No. 84) PB.305p
CCCGTAGGAGTCTGGACCGTGTCTCA

Said system recognizes most of the bacterial pathogens.

All the above outlined probes were provided with the same labelling system as those described in Examples 1 and 2.

All the probes and primers described in the examples are linear DNA oligonucleotides.

Although the complementary sequences lead to suitable probe/primer systems as well, due to a quenching effect on at least the fluorescent marker used in the examples, the probe sequences richer in C than G are preferred.

The sequences that have already been detected and quantified are as follows:

| **already detected sequence:** | **kind of nucleic acid already searched** |
|---|---|
| - FIV | DNA in proviral form and as RNA as it is present in the mature virus |
| - FCoV | as viral RNA |
| - FeLV exogen | as with FIV as provirus and virus |
| - FeLV endogen | DNA (DNA of endogenous FeLV which is present in the genome of many cat species) |
| - CDV | RNA |
| - Borrelia | DNA |
| - Francisella tullarensis | DNA |
| - Ehrlichia risticii | DNA |
| - Ehrlichia phagozytophila | DNA |
| - FSME | RNA |
| - L. monozytogenes | DNA |
| - pan bakt marker | DNA |
| - feline GAPDH, IL-4, IL-10, IL-12, interferone gamma, IL-16 as well as CCR5 | RNA |
| - equine GAPDH, IL-1β, IL-6 as well as iNOS | RNA |

If the target nucleid acid is an RNA, then a reverse transcription step has to be performed prior to the polymerase step.

The same approach as described in the above examples is also suitable for the detection of e.g. canine coronavirus (CCV), transmissible gastroenteritis virus (TGE) and porcine respiratory coronavirus (PRCV).

While there are shown and described specific embodiments of the invention for illustrative reasons, it is to be distinctly understood that the invention is not limited thereto but may be otherwise variously embodied and practiced within the scope of the following claims.

### Annex to the description

(C) STRANDEDNESS: single
(D) TOPOLOGY: linear

(ii) MOLECULE TYPE: other nucleic acid
(A) DESCRIPTION: /desc = "FCoV probe"

(xi) SEQUENCE DESCRIPTION: SEQ ID NO: 3:

TCCATTGTTG GCTCGTCATA GCGGA 25

(2) INFORMATION FOR SEQ ID NO: 4:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "FIV primer"

(xi) SEQUENCE DESCRIPTION: SEQ ID NO: 4:

AGAACCTGGT GATATACCAG AGAC 24

(2) INFORMATION FOR SEQ ID NO: 5:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "FIV primer"

(xi) SEQUENCE DESCRIPTION: SEQ ID NO: 5:

TTGGGTCAAG TGCTACATAT TG 22

(2) INFORMATION FOR SEQ ID NO: 6:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "FIV probe"

(xi) SEQUENCE DESCRIPTION: SEQ ID NO: 6:

TATGCCTGTG GAGGGCCTTC CT 22

(2) INFORMATION FOR SEQ ID NO: 7:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "FIV primer"

(xi) SEQUENCE DESCRIPTION: SEQ ID NO: 7:

GCCTTCTCTG CAAATTTAAC ACCT 24

(2) INFORMATION FOR SEQ ID NO: 8:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "FIV primer"

(xi) SEQUENCE DESCRIPTION: SEQ ID NO: 8:

GATCATATTC TGCTGTCAAT TGCTTT 26

(2) INFORMATION FOR SEQ ID NO: 9:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "FIV probe"

(xi) SEQUENCE DESCRIPTION: SEQ ID NO: 9:

TGCGGCCATT ATTAATGTGG CCATG 25

(2) INFORMATION FOR SEQ ID NO: 10:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "FIV primer"

(xi) SEQUENCE DESCRIPTION: SEQ ID NO: 10:

GATCATATTC TGCTGTCAAT TGCTTT 26

(2) INFORMATION FOR SEQ ID NO: 11:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "FIV primer"

(xi) SEQUENCE DESCRIPTION: SEQ ID NO: 11:

GCCTTCTCTG CAAATTTAAC ACCT 24

(2) INFORMATION FOR SEQ ID NO: 12:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "FIV probe"

(xi) SEQUENCE DESCRIPTION: SEQ ID NO: 12:

CATGGCCACA TTAATAATGG CCGCA 25

(2) INFORMATION FOR SEQ ID NO: 13:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "FCoV primer"

(xi) SEQUENCE DESCRIPTION: SEQ ID NO: 13:

AACAATCACT AGATCCAGAC GTTAGCT 27

(2) INFORMATION FOR SEQ ID NO: 14:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "FCoV primer"

(xi) SEQUENCE DESCRIPTION: SEQ ID NO: 14:

GATTTGATTT GGCAATGCTA GATTT 25

(2) INFORMATION FOR SEQ ID NO: 15:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "FCoV probe"

(xi) SEQUENCE DESCRIPTION: SEQ ID NO: 15:

TCCATTGTTG GCTCGTCATA GCGGA 25

(2) INFORMATION FOR SEQ ID NO: 16:

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "FeLV exogen primer"

(xi) SEQUENCE DESCRIPTION: SEQ ID NO: 16:

ACCTGGGCCC CGGCT 15

(2) INFORMATION FOR SEQ ID NO: 17:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "FeLV exogen primer"

(xi) SEQUENCE DESCRIPTION: SEQ ID NO: 17:

GCGGCCTTGA AACTTCTGCT 20

(2) INFORMATION FOR SEQ ID NO: 18:

(i) SEQUENCE CHARACTERISTICS:

(A) LENGTH: 26 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "FeLV exogen probe"

(xi) SEQUENCE DESCRIPTION: SEQ ID NO: 18:

AGGCCAAGAA CAGTTAAACC CCGGAT 26

(2) INFORMATION FOR SEQ ID NO: 19:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "FeLV endogen primer"

(xi) SEQUENCE DESCRIPTION: SEQ ID NO: 19:

TGTGGGCCCT CTTACGATAA A 21

(2) INFORMATION FOR SEQ ID NO: 20:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "FeLV endogen primer"

(xi) SEQUENCE DESCRIPTION: SEQ ID NO: 20:

CACCGGCCCC CTTCAC 16

(2) INFORMATION FOR SEQ ID NO: 21:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "FeLV endogen probe"

(xi) SEQUENCE DESCRIPTION: SEQ ID NO: 21:

CTGTTCACTC CTCGACAACG GGAGCT                                    26

(2) INFORMATION FOR SEQ ID NO: 22:

     (i) SEQUENCE CHARACTERISTICS:
          (A) LENGTH: 20 base pairs
          (B) TYPE: nucleic acid
          (C) STRANDEDNESS: single
          (D) TOPOLOGY: linear

    (ii) MOLECULE TYPE: other nucleic acid
          (A) DESCRIPTION:   /desc = "FHV-1 primer"

    (xi) SEQUENCE DESCRIPTION: SEQ ID NO: 22:

AGAGGCTAAC GGACCATCGA                                    20

(2) INFORMATION FOR SEQ ID NO: 23:

     (i) SEQUENCE CHARACTERISTICS:
          (A) LENGTH: 19 base pairs
          (B) TYPE: nucleic acid
          (C) STRANDEDNESS: single
          (D) TOPOLOGY: linear

    (ii) MOLECULE TYPE: other nucleic acid
          (A) DESCRIPTION:   /desc = "FHV-1 primer"

    (xi) SEQUENCE DESCRIPTION: SEQ ID NO: 23:

GCCCGTGGTG GCTCTAAAC                                    19

(2) INFORMATION FOR SEQ ID NO: 24:

     (i) SEQUENCE CHARACTERISTICS:
          (A) LENGTH: 35 base pairs
          (B) TYPE: nucleic acid
          (C) STRANDEDNESS: single
          (D) TOPOLOGY: linear

    (ii) MOLECULE TYPE: other nucleic acid
          (A) DESCRIPTION:   /desc = "FHV-1 probe"

    (xi) SEQUENCE DESCRIPTION: SEQ ID NO: 24:

TATATGTGTC CACCACCTTC AGGATCTACT GTCGT                                    35

(2) INFORMATION FOR SEQ ID NO: 25:

     (i) SEQUENCE CHARACTERISTICS:
          (A) LENGTH: 23 base pairs
          (B) TYPE: nucleic acid
          (C) STRANDEDNESS: single
          (D) TOPOLOGY: linear

    (ii) MOLECULE TYPE: other nucleic acid
          (A) DESCRIPTION:   /desc = "CDV primer"

(xi) SEQUENCE DESCRIPTION: SEQ ID NO: 25:

GGAAGCCTTG ATGATAGCAC TGA 23

(2) INFORMATION FOR SEQ ID NO: 26:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "CDV primer"

(xi) SEQUENCE DESCRIPTION: SEQ ID NO: 26:

GCCGAAAGAA TATCCCCAGT T 21

(2) INFORMATION FOR SEQ ID NO: 27:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "CDV probe"

(xi) SEQUENCE DESCRIPTION: SEQ ID NO: 27:

TCTGGCGAAG ATTATTCCGA AGGAAATGCT 30

(2) INFORMATION FOR SEQ ID NO: 28:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Borrelia primer"

(xi) SEQUENCE DESCRIPTION: SEQ ID NO: 28:

GGGAAGCAGA TTTGTTTGAC A 21

(2) INFORMATION FOR SEQ ID NO: 29:

(i) SEQUENCE CHARACTERISTICS:
(A) LENGTH: 30 base pairs
(B) TYPE: nucleic acid
(C) STRANDEDNESS: single

(D) TOPOLOGY: linear

(ii) MOLECULE TYPE: other nucleic acid
(A) DESCRIPTION: /desc = "Borrelia primer"

(xi) SEQUENCE DESCRIPTION: SEQ ID NO: 29:

ATAGAGCAAC TTACAGACGA AATTAATAGA 30

(2) INFORMATION FOR SEQ ID NO: 30:

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Borrelia probe"

(xi) SEQUENCE DESCRIPTION: SEQ ID NO: 30:

ATGTGCATTT GGTTATATTG AGCTTGATCA GCAA 34

(2) INFORMATION FOR SEQ ID NO: 31:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Bartonella henselae
primer"

(xi) SEQUENCE DESCRIPTION: SEQ ID NO: 31:

AAGGTGCTAA TCCATTTGCA TGTATT 26

(2) INFORMATION FOR SEQ ID NO: 32:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Bartonella henselae
primer"

(xi) SEQUENCE DESCRIPTION: SEQ ID NO: 32:

AGTAACATTT TTAGGCATGC TTCATTA 27

(2) INFORMATION FOR SEQ ID NO: 33:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Bartonella henselae probe"

(xi) SEQUENCE DESCRIPTION: SEQ ID NO: 33:

AGCTGGTCCC CAAAGGCATG CAA 23

(2) INFORMATION FOR SEQ ID NO: 34:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Francisella tularensis
primer"

(xi) SEQUENCE DESCRIPTION: SEQ ID NO: 34:

TTGGGCAAAT CTAGCAGGTC A 21

(2) INFORMATION FOR SEQ ID NO: 35:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Francisella tularensis
primer"

(xi) SEQUENCE DESCRIPTION: SEQ ID NO: 35:

ATCTGTAGTC AACACTTGCT TGAACA 26

(2) INFORMATION FOR SEQ ID NO: 36:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Francisella tularensis

probe"

(xi) SEQUENCE DESCRIPTION: SEQ ID NO: 36:

AAGACCACCA CCAACATCCC AAGCA 25

(2) INFORMATION FOR SEQ ID NO: 37:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Ehrlichia risticii primer"

(xi) SEQUENCE DESCRIPTION: SEQ ID NO: 37:

AGTTATTCCC TACTACCAGG CAAGTTC 27

(2) INFORMATION FOR SEQ ID NO: 38:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Ehrlichia risticii primer"

(xi) SEQUENCE DESCRIPTION: SEQ ID NO: 38:

AACGGAATCA GGGCTGCTT 19

(2) INFORMATION FOR SEQ ID NO: 39:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Ehrlichia risticii probe"

(xi) SEQUENCE DESCRIPTION: SEQ ID NO: 39:

ACGCACCCGT CTGCCACGGG A 21

(2) INFORMATION FOR SEQ ID NO: 40:

(i) SEQUENCE CHARACTERISTICS:
(A) LENGTH: 27 base pairs

(B) TYPE: nucleic acid
(C) STRANDEDNESS: single
(D) TOPOLOGY: linear

(ii) MOLECULE TYPE: other nucleic acid
(A) DESCRIPTION: /desc = "Ehrlichia phagocytophila
primer"

(xi) SEQUENCE DESCRIPTION: SEQ ID NO: 40:

CCATTTCTAG TGGCTATCCC ATACTAC 27

(2) INFORMATION FOR SEQ ID NO: 41:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Ehrlichia phagocytophilia
primer"

(xi) SEQUENCE DESCRIPTION: SEQ ID NO: 41:

TCGAACGGAT TATTCTTTAT AGCTTG 26

(2) INFORMATION FOR SEQ ID NO: 42:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Ehrlichia phagocytophila
probe"

(xi) SEQUENCE DESCRIPTION: SEQ ID NO: 42:

CCTATGCATT ACTCACCCGT CTGCCACT 28

(2) INFORMATION FOR SEQ ID NO: 43:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "FSME primer"

(A) DESCRIPTION: /desc = "L. monocytogens primer"

(xi) SEQUENCE DESCRIPTION: SEQ ID NO: 47:

GCCCAAATAG TGTCACCGCT 20

(2) INFORMATION FOR SEQ ID NO: 48:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "L. monocytogenes probe"

(xi) SEQUENCE DESCRIPTION: SEQ ID NO: 48:

CAAACTACAC AAGCAACTAC ACCTGCGCC 29

(2) INFORMATION FOR SEQ ID NO: 49:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Feline GAPDH primer"

(xi) SEQUENCE DESCRIPTION: SEQ ID NO: 49:

GCCGTGGAAT TTGCCGT 17

(2) INFORMATION FOR SEQ ID NO: 50:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Feline GAPDH primer"

(xi) SEQUENCE DESCRIPTION: SEQ ID NO: 50:

GCCATCAATG ACCCCTTCAT 20

(2) INFORMATION FOR SEQ ID NO: 51:

(i) SEQUENCE CHARACTERISTICS:
(A) LENGTH: 37 base pairs

(B) TYPE: nucleic acid
(C) STRANDEDNESS: single
(D) TOPOLOGY: linear

(ii) MOLECULE TYPE: other nucleic acid
(A) DESCRIPTION: /desc = "Feline GAPDH probe"

(xi) SEQUENCE DESCRIPTION: SEQ ID NO: 51:

CTCAACTACA TGGTCTACAT GTTCCAGTAT GATTCCA 37

(2) INFORMATION FOR SEQ ID NO: 52:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Feline IL-4 primer"

(xi) SEQUENCE DESCRIPTION: SEQ ID NO: 52:

GCATGGAGCT GACCGTCAT 19

(2) INFORMATION FOR SEQ ID NO: 53:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Feline IL-4 primer"

(xi) SEQUENCE DESCRIPTION: SEQ ID NO: 53:

CGGTTGTGGC TCTGCAGA 18

(2) INFORMATION FOR SEQ ID NO: 54:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Feline IL-4 probe"

(xi) SEQUENCE DESCRIPTION: SEQ ID NO: 54:

TGGCAGCCCC TAAGAACACA AGTGACAA 28

(2) INFORMATION FOR SEQ ID NO: 55:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Feline IL-10 primer"

(xi) SEQUENCE DESCRIPTION: SEQ ID NO: 55:

TGCACAGCAT ATTGTTGACC AG 22

(2) INFORMATION FOR SEQ ID NO: 56:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Feline IL-10 primer"

(xi) SEQUENCE DESCRIPTION: SEQ ID NO: 56:

ATCTCGGACA AGGCTTGGC 19

(2) INFORMATION FOR SEQ ID NO: 57:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Feline IL-10 probe"

(xi) SEQUENCE DESCRIPTION: SEQ ID NO: 57:

ACCCAGGTAA CCCTTAAAGT CCTCCAGCA 29

(2) INFORMATION FOR SEQ ID NO: 58:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Feline IL-12 primer"

(xi) SEQUENCE DESCRIPTION: SEQ ID NO: 58:

TGGCTTCAGT TGCAGGTTCT T                                                21

(2) INFORMATION FOR SEQ ID NO: 59:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION:   /desc = "Feline IL-12 primer"

(xi) SEQUENCE DESCRIPTION: SEQ ID NO: 59:

TGGACGCTAT TCACAAGCTC A                                                21

(2) INFORMATION FOR SEQ ID NO: 60:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid
(A) DESCRIPTION:   /desc = "Feline IL-12 probe"

(xi) SEQUENCE DESCRIPTION: SEQ ID NO: 60:

CGGTTTGATG ATGTCCCTGA TGAAGAAGCT                                       30

(2) INFORMATION FOR SEQ ID NO: 61:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION:  /desc = "Feline IFN-gamma primer"

(xi) SEQUENCE DESCRIPTION: SEQ ID NO: 61:

TGGTGGGTCG CTTTTCGTAG                                                  20

(2) INFORMATION FOR SEQ ID NO: 62:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Feline IFN-gamma primer"

(xi) SEQUENCE DESCRIPTION: SEQ ID NO: 62:

GAAGGAGACA ATTTGGCTTT GAA 23

(2) INFORMATION FOR SEQ ID NO: 63:

(i) SEQUENCE CHARACTERISTICS:
(A) LENGTH: 39 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Feline IFN-gamma probe"

(xi) SEQUENCE DESCRIPTION: SEQ ID NO: 63:

CATTTTGAAG AACTGGAAAG AGGAGAGTGA TAAAACAAT 39

(2) INFORMATION FOR SEQ ID NO: 64:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Feline IL-16 primer"

(xi) SEQUENCE DESCRIPTION: SEQ ID NO: 64:

ACTGATTCTA CAAACTCGGC TTCTG 25

(2) INFORMATION FOR SEQ ID NO: 65:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Feline IL-16 primer"

(xi) SEQUENCE DESCRIPTION: SEQ ID NO: 65:

ATTTCATCTC CCGGCTGGAT 20

(2) INFORMATION FOR SEQ ID NO: 66:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Feline IL-16 probe"

(xi) SEQUENCE DESCRIPTION: SEQ ID NO: 66:

TTCCGAGGCT GCCCCTTTGA AAATCCTGTT CA 32

(2) INFORMATION FOR SEQ ID NO: 67:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Feline CCR5 primer"

(xi) SEQUENCE DESCRIPTION: SEQ ID NO: 67:

TCGGAGCCCT GCCAGAA 17

(2) INFORMATION FOR SEQ ID NO: 68:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Feline CCR5 primer"

(xi) SEQUENCE DESCRIPTION: SEQ ID NO: 68:

GTAGAGCGGA GGCAGGAGTC T 21

(2) INFORMATION FOR SEQ ID NO: 69:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Feline CCR5 probe"

(xi) SEQUENCE DESCRIPTION: SEQ ID NO: 69:

CCGACGTGAG GCAAATCGCA GC 22

(2) INFORMATION FOR SEQ ID NO: 70:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Equine GAPDH primer"

(xi) SEQUENCE DESCRIPTION: SEQ ID NO: 70:

AAGTGGATAT TGTCGCCATC AAT 23

(2) INFORMATION FOR SEQ ID NO: 71:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Equine GAPDH primer"

(xi) SEQUENCE DESCRIPTION: SEQ ID NO: 71:

AACTTGCCAT GGGTGGAATC 20

(2) INFORMATION FOR SEQ ID NO: 72:

(i) SEQUENCE CHARACTERISTICS:
(A) LENGTH: 38 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Equine GAPDH probe"

(xi) SEQUENCE DESCRIPTION: SEQ ID NO: 72:

ACCTCAACTA CATGGTCTAC ATGTTTCAGT ATGATTCC 38

(2) INFORMATION FOR SEQ ID NO: 73:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Equine IL-1beta primer"

(C) STRANDEDNESS: single
(D) TOPOLOGY: linear

(ii) MOLECULE TYPE: other nucleic acid
(A) DESCRIPTION: /desc = "Equine IL-6 primer"

(xi) SEQUENCE DESCRIPTION: SEQ ID NO: 77:

CCAGATTGGA AGCATCCGTC 20

(2) INFORMATION FOR SEQ ID NO: 78:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Equine IL-6 probe"

(xi) SEQUENCE DESCRIPTION: SEQ ID NO: 78:

ATCTCTGCCC TGAAAAATGA GATGTGTAAC AATTT 35

(2) INFORMATION FOR SEQ ID NO: 79:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Equine iNOS primer"

(xi) SEQUENCE DESCRIPTION: SEQ ID NO: 79:

GGATGACTTT CGAGGACATG C 21

(2) INFORMATION FOR SEQ ID NO: 80:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Equine iNOS primer"

(xi) SEQUENCE DESCRIPTION: SEQ ID NO: 80:

GGGCCCTCTG GTCATACTTT T 21

(2) INFORMATION FOR SEQ ID NO: 81:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Equine iNOS probe"

(xi) SEQUENCE DESCRIPTION: SEQ ID NO: 81:

AAGCGACCTC CCCATTGGCC T 21

(2) INFORMATION FOR SEQ ID NO: 82:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Pan Bakt primer"

(xi) SEQUENCE DESCRIPTION: SEQ ID NO: 82:

GGATGATCAG CCACACTGGA 20

(2) INFORMATION FOR SEQ ID NO: 83:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Pan Bakt primer"

(xi) SEQUENCE DESCRIPTION: SEQ ID NO: 83:

CCAATATTCC TCACTGCTGC C 21

(2) INFORMATION FOR SEQ ID NO: 84:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Pan Bakt probe"

(xi) SEQUENCE DESCRIPTION: SEQ ID NO: 84:

CCCGTAGGAG TCTGGACCGT GTCTCA 26

(2) INFORMATION FOR SEQ ID NO: 85:

(i) SEQUENCE CHARACTERISTICS:
(A) LENGTH: 272 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Feline Corona Virus

(xi) SEQUENCE DESCRIPTION: SEQ ID NO: 85:

GGCAACCCGA TGTCTAAAAC TGGTCTTTCC GAGGAATTAC GGGTCATCGC GCTGCCTACT 60

CTTGTACAGA ATGGTAAGCA CGTGTAATAG GAGGTACAAG CAACCCTATT GCATATTAGG 120

AAGTTTAGAT TTGATTTGGC AATGCTAGAT TTAGTAATTT AGAGAAGTTT AAAGATCCGC 180

TATGACGAGC CAACAATGGA AGAGCTAACG TCTGGATCTA GTGATTGTTT AAAATGTAAA 240

ATTGTTTGAA AATTTTCCTT TTGATAGTGA TG 272

(2) INFORMATION FOR SEQ ID NO: 86:

(i) SEQUENCE CHARACTERISTICS:
(A) LENGTH: 505 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Feline Immunodeficiency Virus

(xi) SEQUENCE DESCRIPTION: SEQ ID NO: 86:

GAGAACCTGG TGATATACCA GAGACTTTAG ATCAACTAAG GTTGGTTATT TGCGATTTAC 60

AAGAAAGAAG AGAAAAATTT GGATCTAGCA AAGAAATTGA TATGGCAATT GTGACATTAA 120

AAGTCTTTGC GGTAGCAGGA CTTTTAAATA TGACGGTGTC TACTGCTGCT GCAGCTGAAA 180

ATATGTATTC TCAAATGGGA TTAGACACTA GGCCATCTAT GAAAGAAGCA GGTGGAAAAG 240

AGGAAGGCCC TCCACAGGCA TATCCTATTC AAACAGTAAA TGGAGTACCA CAATATGTAG 300

CACTTGACCC AAAAATGGTG TCCATTTTTA TGGAAAAGGC AAGAGAAGGA CTAGGAGGTG 360

AGGAAGTTCA ACTATGGTTT ACTGCCTTCT CTGCAAATTT AACACCTACT GACATGGCCA 420

CATTAATAAT GGCCGCACCA GGGTGCGCTG CAGATAAAGA AATATTGGAT GAAAGCTTAA 480

AGCAACTGAC AGCAGAATAT GATCG 505

SEQUENCE LISTING

(1) GENERAL INFORMATION:

(i) APPLICANT:
(A) NAME: Lutz Hans
(B) STREET: Im Oberdorf
(C) CITY: Ruedlingen
(E) COUNTRY: Switzerland
(F) POSTAL CODE (ZIP): 8455

(ii) TITLE OF INVENTION: Quantitative polymerase chain reaction
fluorogenic real-time detection system

(iii) NUMBER OF SEQUENCES: 86

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "FCoV primer"

(xi) SEQUENCE DESCRIPTION: SEQ ID NO: 1:

GATTTGATTT GGCAATGCTA GATTT 25

(2) INFORMATION FOR SEQ ID NO: 2:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "FCoV primer"

(xi) SEQUENCE DESCRIPTION: SEQ ID NO: 2:

AACAATCACT AGATCCAGAC GTTAGCT 27

(2) INFORMATION FOR SEQ ID NO: 3:

(i) SEQUENCE CHARACTERISTICS:
(A) LENGTH: 25 base pairs
(B) TYPE: nucleic acid

(xi) SEQUENCE DESCRIPTION: SEQ ID NO: 43:

TCCAGTGGAG CTGGAGTTGA G 21

(2) INFORMATION FOR SEQ ID NO: 44:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "FSME primer"

(xi) SEQUENCE DESCRIPTION: SEQ ID NO: 44:

TCCTCCATTC AGGGTTGACA A 21

(2) INFORMATION FOR SEQ ID NO: 45:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "FSME probe"

(xi) SEQUENCE DESCRIPTION: SEQ ID NO: 45:

AATAAGCTTG AACTACCTGG GCTGGCA 27

(2) INFORMATION FOR SEQ ID NO: 46:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "L. monocytogenes primer"

(xi) SEQUENCE DESCRIPTION: SEQ ID NO: 46:

AAGCTGCACC TGTTGCAGAA 20

(2) INFORMATION FOR SEQ ID NO: 47:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 73:

GCAGTACCCG ACACCAGTGA 20

(2) INFORMATION FOR SEQ ID NO: 74:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Equine IL-lbeta primer"

(xi) SEQUENCE DESCRIPTION: SEQ ID NO: 74:

TTTTGGGCCA TCCTCCTCA 19

(2) INFORMATION FOR SEQ ID NO: 75:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Equine IL-lbeta probe"

(xi) SEQUENCE DESCRIPTION: SEQ ID NO: 75:

TCATTCTCAT TGCCGCTGCA GTAAGTCAT 29

(2) INFORMATION FOR SEQ ID NO: 76:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Equine IL-6 primer"

(xi) SEQUENCE DESCRIPTION: SEQ ID NO: 76:

AGCACATTAA GTACATCCTC GGC 23

(2) INFORMATION FOR SEQ ID NO: 77:

(i) SEQUENCE CHARACTERISTICS:
(A) LENGTH: 20 base pairs
(B) TYPE: nucleic acid

### Literature References

Adler, K., Buzby, P., Bobrow, M., Erickson, T. Rapid DNA detection using a fluorescein-antifluorescein-based (FAF) ELOSA, 1992. DuPont Biotech Update, 7:13-15.

Allenspach, K., Amacher, M., Leutenegger, C., Hottiger, M., Hofmann-Lehmann, R., Hubscher, U., Pistello, M., Lutz, H. Quantification of proviral FIV DNA using competitive PCR, 1996. SAT 138(2):87-92.

Bachmann, M.H., Mathiason-Dubard, C., Learn, G.H., Rodrigo, A.G., Sodora, D.L., Mazzetti, P., Hoover, E.A., Mullins, J.I. Genetic diversity of feline immunodeficiency virus: dual infection, recombination, and distinct evolutionary rates among envelope sequence clades, 1997. J. Virol. 1997;71:4241-53.

Birnboim, H.C. and Doly, J. (1979) A rapid alkaline extraction procedure for screening recombinant plasmid DNA. Nucleic acids research 7(6), 1513-1523.

Bridgen, A., Duarte, M., Tobler, K., Laude, H. and Ackermann, M. (1993) Sequence determination of the nucleocapsid protein gene of the porcine epidemic diarrhoea virus confirms that this virus is a coronavirus related to human coronavirus 229E and porcine transmissible gastroenteritis virus. Journal of General Virology 74(Pt 9), 1795-804.

Cammarota, G., Da Prato, L., Nicoletti, E., Matteucci, D., Bendinelli, M., Pistello, M. Quantitation of feline immunodeficiency proviruses in doubly infected cats using competitive PCR and a fluorescence-based RFLP, 1996. J. Virol. Methods 62(1):21-31.

Chomczynski P. and Sacchi N. Single-step method of RNA isolation by acid guanidinium thiocyanatephenol-chloroform extraction, 1987. Anal. Biochem. 162(1):156-159.

Clementi, M., Menzo, S., Bagnarelli, P., Manzin, A., Valenza, A., and Varaldo, P.E. Quantitative PCR and RT-PCR in virology, 1993. PCR Methods & Applications 2(3):191-196.

Diehl, L.J., Mathiason-DuBard, C.K., O'Neil, L.L., and Hoover, E.A. Longitudinal assessment of feline immunodeficiency virus kinetics in plasma by use of a quantitative competitive reverse transcriptase PCR, 1995. J. Virol. 69(4):2328-2332.

Foley, J.E., Poland, A., Carlson, J. and Pedersen, N.C. (1997) Patterns of feline coronavirus infection and fecal shedding from cats in multiple-cat environments. Journal of the American Veterinary Medical Association 210(9), 1307-12.

Förster V.Th. Zwischenmolekulare Energiewanderung und Fluoreszenz, 1948. Ann Phy 2:55-75.

Gamble, D.A., Lobbiani, A., Gramegna, M., Moore, L.E. and Colucci, G. (1997) Development of a nested PCR assay for detection of feline infectious peritonitis virus in clinical specimens. Journal of Clinical Microbiology 35(3), 673-5.

Gardner, M.B. Simian and feline immunodeficiency viruses: animal lentivirus models for evaluation of AIDS vaccines and antiviral agents, 1991. Antiviral. Res. 15(4):267-286.

Gelfand, "Taq DNA Polymerase" in PCR Technology; Principles and Applications for DAN Amplification, Erlich, Ed., Stockton Press, N.Y. (1989), Chapter 2

Herrewegh, A.A.P.M., de Groot, R.J., Cepica, A., Egberink, H.F., Horzinek, M.C. and Rottier, P.J. (1995) Detection of feline coronavirus RNA in feces, tissues, and body fluids of naturally infected cats by reverse transcriptase PCR. Journal of Clinical Microbiology 33(3), 684-9.

Holland, P.M., Abramson, R.D., Watson, R., and Gelfand, D.H. Detection of specific polymerase chain reaction product by utilizing the 5'-3' exonuclease activity of Thermus aquaticus DNA polymerase, 1991. Proc. Natl. Acad. Sci. 88(16):7276-7280.

Johnson, C.M., Selleseth, D.W., Ellis, M.N., Childers, T.A., Tompkins, M.B., Tompkins, W.A. Reduced provirus burden and bnhanced humoral immune function in AZT-treated SCID-feline mice inoculated with feline immunodeficiency virus, 1995. Vet. Immunol. Immunopathol. 46:169-80.

Kakinuma, S., Motokawa, K., Hohdatsu, T., Yamamoto, J.K., Koyama, H., and Hashimoto, H. Nucleotide sequence of feline immunodeficiency virus: classification of Japanese isolates into two subtypes which are distinct from non-Japanese subtypes, 1995. J. Virol. 69(6):3639-3646.

Kinoshita, T., Imamura, J., Nagai, H. and Shimotohno, K. (1992) Quantification of gene expression over a wide range by the polymerase chain reaction. Analytical Biochemistry 206(2), 231-5.

Leutenegger, C.M., Hofmann-Lehmann, R., Holznagel, E., Cuisinier, A.M., Wolfensberger, C., Duquesne, V., Cronier, J., Allenspach, K., Aubert, A., Ossent, P., Lutz, H, 1998. Partial protection by vaccination with recombinant feline immunodeficiency virus surface glycoproteins. AIDS Res. Hum. Retroviruses 14:275-83.

Li, X. and Scott, F.W. (1994) Detection of feline coronaviruses in cell cultures and in fresh and fixed feline tissues using polymerase chain reaction. Veterinary Microbiology 42(1), 65-77.

Livak, K., Marmaro, J., Flood, S. Guidelines for designing TaqMan fluorogenic probes for 5' nuclease assays, 1995. PE Applied Biosystems, Research News.

Menzo, S., Bagnarelli, P., Giacca, M., Manzin, A., Varaldo, P.E., and Clementi, M. Absolute quantification of viremia in human immunodeficiency virus infection by competitive reverse transcription and polymerase chain reaction, 1992. J. Clin. Microbiol. 30(7):1752-1757.

Nagano, M. and Kelly, P.A. (1994) Tissue distribution and regulation of rat prolactin receptor gene expression. Quantitative analysis by polymerase chain reaction. Journal of Biological Chemistry 269(18), 13337-45.

Pang, J., Modlin, J., Yolken, R. Use of modified nucleotides and uracil-DNA glycosylase (UNG) for the control of contamination in the PCR-based amplification of RNA, 1992. Mol Cell Probes 6(3):251-256.

Pedersen, N.C., Ho, E.W., Brown, M.L., and Yamamoto, J.K. Isolation of a T-lymphotropic virus from domestic cats with an immunodeficiency-like syndrome, 1987. Science 235(4790):790-793.

Pistello, M., Mendo, S., Giogi, M., Da Prato, L., Cammarota, G., Clementi, M., Bendinelli, M. Competitive polymerase chain reaction for quantitating feline immunodeficiency virus load in infected cat tissues, 1994. Mol. Cel. Probes 8:229-234.

Saiki et al., 1988, Science 239:487

Sambrook, J., Fritsch, E.F. and Maniatis, T. (1989) . Molecular cloning - a laboratory manual (second edition).

Siebert, P.D. and Larrick, J.W. Competitive PCR, 1992. Nature 359(6395):557-558.

Simmonds, P., Balfe, P., Peutherer, J.F., Ludlam, C.A., Bishop, J.O., Leigh Brown, A.J., Human immunodeficiency virus-infected individuals contain provirus in samll numbers in peripheral mononuclear cells at low copy numbers, 1990. J. Virol. 64:864-872.

Southern, E.M. Detection of specific sequences among DNA fragments separated by gel electrophoresis, 1975. J. Molec. Biol. 98(3):503-517.

Sundfors, C., Collan, Y. Basics of quantitative polymerase chain reaction: 2. Electrophoresis and quantitation of polymerase chain reaction products, 1996. Electrophoresis 17(1):44-48.

Tomonaga, K. and Mikami, T, 1996. Detection of feline immunodeficiency virus transcripts by quantitative reverse transcription-polymerase chain reaction. Vet. Microbiol. 48(3-4):337-344.

Vahlenkamp, T.W., Egberink, H.F., van Eijk, M.J., Slotboom-Kamphorst, A.M., Verschoor, E.J., Horzinek, M.C., and De Ronde, A, 1995. Competitive reverse transcription-polymerase chain reaction for quantification of feline immunodeficiency virus. J. Virol. Methods 52(3):335-346.

Wang, A.M., Doyle, M.V. and Mark, D.F. (1989) Quantitation of mRNA by the polymerase chain reaction [published erratum appears in Proc Natl Acad Sci U S A 1990 Apr;87(7):2865]. Proceedings of the National Academy of Sciences of the United States of America 86(24), 9717-21.

Zhang, LQ., Simmonds, P., Ludlam, C.A., Leigh Brown, A.J. Detection, quantification and sequencing of HIV-1 from plasma of seropositive individuals and from factor VIII concentrates, 1991. AIDS 5:675-681.

## Claims

1. A method for identification and quantification of at least one pathogen in a sample, said method being performed on the DNA level, and said method comprising:
(A) providing to a PCR assay containing said sample, at least one labeled oligonucleotide probe containing a sequence complementary to a region of the target nucleic acid, wherein said labeled oligonucleotide probe anneals within the target nucleic acid sequence bounded by the oligonucleotide primer of step (B);
(B) providing at least one set of oligonucleotide primers, wherein a first primer contains a sequence complementary to a region in one strand of the target nucleic acid sequence and primes the synthesis of a complementary DNA strand, and a second primer contains a sequence complementary to a region in a second strand of the target nucleic acid sequence and primes the synthesis of a complementary DNA strand; and wherein each oligonucleotide primer is selected to anneal to its complementary template upstream of any labeled oligonucleotide annealed to the same nucleic acid strand;
(C) amplifying the target nucleic acid sequence employing a nucleic acid polymerase having 5' to 3' nuclease activity as a template-dependent polymerizing agent under conditions which are permissive for PCR cycling steps of (i) annealing of primers and labeled oligonucleotide to a template nucleic acid sequence contained within the target region, and (ii) extending the primer, wherein said nucleic acid polymerase synthesizes a primer extension product while the 5' to 3' nuclease activity of the nucleic acid polymerase simultaneously releases labeled fragments from the annealed duplexes comprising labeled oligonucleotide and its complementary template nucleic acid sequences, thereby creating detectable labeled fragments; and
(D) detecting and/or measuring the release or interference of labeled fragments to determine the presence or absence of a target sequence in the sample, **characterized in that** the probe has a length and/or a content of G and/or C sufficient to provide a hybridization force strong enough to ensure hybridization in the presence of just the desired number of mismatches, and in that said probe and primers define an amplicon of a length of at most 300 bp, preferably at most 250 bp, most preferably between 100 and 225 bp.

2. The method of claim 1, characterized in that the probe has a length of at least 20 nucleic acids and a hybridization force of at least 50 in the presence of the highest amount of mismatches.

3. The method of claim 1 or 2, characterized in that the at least one probe has a length and G and/or G content sufficient to ensure that the mismatches to be covered by said probe reduce the hybridization force to an extent of not more but 20 %, much preferably not more but 15 %, whereby the minimal hybridization force has to be 50.

4. The method of anyone of claims 1 to 3, characterized in that the probe has a melting temperature which is at least about 5, preferably at least about 6°C, most preferably about 10°C higher than the one of the primers.

5. The method of anyone of claims 1 to 4, characterized in that the probe has a melting temperature of from about 67 to about 71°C, preferably of about 69°C.

6. The method of anyone of claims 1 to 5, characterized in that the primers have a melting temperature of from about 57 to about 61°C, in particular about 59°C.

7. The method of anyone of claims 1 to 6, characterized in that the primers preferably have a length of 18 to 22 bp and the probes of 20 to 40 bp, preferably of 24 to 30 bp.

8. The method of anyone of claims 1 to 7, characterized in that the distance between primer and probe is at least 1 bp, and at most the length of the amplicon minus the two primers and the probe, preferably 100 bp or less.

9. The method of anyone of claims 1 to 8, characterized in that the cycle is performed with two temperatures, said temperature being the Tₘ of the primers and the denaturation temperature.

10. The method of claim 9, characterized in that the denaturation temperature is from about 85 to about 95°C.

11. The method of anyone of claims 1 to 10, characterized in that the probe is selected from a region where an intron has been spliced off, whereby the probe comprises from 30 to 70 % of a first exon and from 70 to 30 % of a second exon, preferably about 50 % of each exon.

12. The method of claim 1 wherein the probes and the forward and revers primers are selected from respective combinations of those of SEQ ID Nos 1 to 3, 7 to 9, 10 to 12, 13 to 15, 16 to 18, 19 to 21, 22 to 24, 25 to 27, 28 to 30, 31 to 33, 34 to 36, 37 to 39, 40 to 42, 43 to 45, 46 to 48, 49 to 51, 52 to 54, 55 to 57, 58 to 60, 61 to 63, 64 to 66, 67 to 69, 70 to 72, 73 to 75, 76 to 78, 79 to 81, 82 to 84.

13. A nucleotide sequence characterized in that it has at least 80 %, preferably 85 % identity with a pathogen, and a hybridization strength in the case of at least 80 % identity of at least 50, in particular a nucleotide sequence suitable for use as probe in the method of anyone of claims 1 to 12.

14. The nucleotide sequence of claim 13, characterized in that it has a length of from 20 to 40 bp, preferably 20 to 30 bp.

15. The nucleotide sequence of claim 13 or 14, characterized in that it has a melting temperature of from about 67 to about 71°C, preferably of about 69°C.

16. The nucleotide sequence of anyone of claims 13 to 15, characterized in that it is selected from a sequence spanning two exons with from 30 to 70 % of the bp from 1 exon and from 70 to 30 % of the bp from the second exon, preferably about 50 % from each exon.

17. The nucleotide sequences of SEQ ID Nos 1 to 3, 7 to 9, 10 to 12, 13 to 15, 16 to 18, 19 to 21, 22 to 24, 25 to 27, 28 to 30, 31 to 33, 34 to 36, 37 to 39, 40 to 42, 43 to 45, 46 to 48, 49 to 51, 52 to 54, 55 to 57, 58 to 60, 61 to 63, 64 to 66, 67 to 69, 70 to 72, 73 to 75, 76 to 78, 79 to 81, 82 to 84.

18. A kit for detecting and quantifying pathogens, characterized in that it comprises at least one probe being a nucleotide sequence of one of claims 13 to 17 and at least one set of corresponding primers in separate containers, whereby the probe and corresponding primers define an amplicon of a length of at most 300 bp, preferably 250 bp, most preferably between 100 and 225 bp.

19. The kit of claim 18, characterized in that the probe is unlabeled, and specific labeling reagents are also provided as separate component in the kit.

20. A method for preparing a nucleotide sequence according to anyone of claims 13 to 17, said sequence being suitable as probe for the detection of a specific pathogen, characterized in that sequences of mutants and/or isolates are compared, in that at least one sequence with the desired conservation enabling identification of the desired number of pathogenic mutants and/or isolates and distinction from non-desired and/or non-pathogenic mutants and/or isolates is determined, whereby said at least one sequence has the needed length and hybridization force, and in that said nucleotide sequence is prepared.

21. The method according to claim 20, characterized in that the at least one nucleotide sequence is provided with a label allowing the distinction between the least two of the following states of the probe: not hybridized, hybridized and released/destroyed.
